# EUROPÄISCHE PATENTSCHRIFT

(11) **EP 2 712 677 B1**
(45) Veröffentlichungstag und Bekanntmachung des Hinweises auf die Patenterteilung: **08.03.2017**
(21) Anmeldenummer: 13183217.2
(22) Anmeldetag: 05.09.2013
(51) Int. Cl.: B01L 3/00, G01N 33/53

(54) **Vorrichtung zur Durchführung einer biochemischen Analyse, insbesondere im Weltraum**
Device for biochemical analysis in particular in outer space
Appareil pour l'analyse biochimique en particulier dans l'espace

(30) Priorität: 01.10.2012 DE 102012109317
(43) Veröffentlichungstag der Anmeldung: 02.04.2014
(73) Patentinhaber: Airbus DS GmbH, 82024 Taufkirchen (DE)
(72) Erfinder: Kern, Peter, 88682 Salem (DE); Janson, Jessica, 99880 Wahlwinkel (DE); Szydzik, Crispin, Napoleons, Victoria 3352 (AU)
(74) Vertreter: Daub, Thomas

(56) Entgegenhaltungen:
- EP-A2- 0 144 162
- WO-A2-2005/069766
- WO-A2-2010/017381
- US-A- 3 969 190
- US-A1- 2010 311 177
- US-A1- 2011 071 055

## Beschreibung

### Stand der Technik

Die Erfindung betrifft eine Vorrichtung nach dem Oberbegriff des Anspruchs 1.

Eine häufig genutzte biochemische Analysemethode zum qualitativen und/oder quantitativen Nachweis eines Analyten in einer Probe ist von den als Immunoassays bezeichneten Verfahren gebildet. Immunoassays beruhen auf dem Funktionsprinzip einer selektiven Bindung eines Analyten in der Probe durch ein analytspezifisches Paar von Fängerantikörpern (capture-Antikörpern, cAB) und Detektionsantikörpern (detection-Antikörper, dAB), wobei letztere eine Markersubstanz an sich gebunden tragen oder zur Bindung der Markersubstanz im Verlauf des Verfahrens vorgesehen sind. Die Fängerantikörper sind dazu vorgesehen, den Analyten an einer festen Position, beispielsweise eine Oberfläche, an der die Fängerantikörper gebunden sind, oder an Trägerteilchen für die Fängerantikörper, zu fixieren. Der Detektionsantikörper bindet selektiv an den Analyten oder an den Fängerantikörper. Über die Markersubstanz wird ein messbares Signal, das zu einer Ermöglichung einer Detektion eines entstandenen Analytkomplexes aus Analyt, Fängerantikörper und Detektionsantikörper vorgesehen ist, erzeugt. In den als so genannte Enzyme-linked Immunosorbent Assays (ELISA's) bezeichneten Immunoassays wird der Analyt mittels eines Enzyms als Markersubstanz markiert, das an dem Detektionsantikörper fixiert vorliegen oder in einem weiteren Reaktionsschritt an den Detektionsantikörper gebunden wird, wobei in einer nachfolgenden enzymkatalysierten Reaktion eine chromogene oder eine lumineszente Verbindung, beispielsweise eine chemo-, elektro-, biolumineszente oder fluoreszente Verbindung, aus einem zugegebenen Substrat erzeugt wird, die mit optischen Methoden detektiert werden kann. Um eine Sättigung eines Signals der chromogenen oder luminszenten Verbindung zu vermeiden, wird nach einer vorgegebenen Zeitspanne ein Stoppermittel zur Unterbrechung der enzymkatalysierten Reaktion hinzugegeben. Das Stoppermittel kann die Unterbrechung beispielsweise durch eine Veränderung eines pH-Werts bewirken, wobei über die Veränderung des pH-Werts häufig ein entstandenes Produkt aus der Reaktion des Substrats mit dem Enzym in der Art eines pH-Indikators sichtbar gemacht wird. Im Fall der sogenannten Radio-Immunoassays (RIA's) werden radioaktive Substanzen als an den Detektionsantikörper gebundene Markersubstanzen verwendet, wobei eine quantitative Bestimmung des Analyten über eine Messung der Radioaktivität erfolgt. Insbesondere für eine präzise quantitative Bestimmung des Analyten ist eine sorgfältige Durchmischung der Probe, der Fängerantikörper, der Detektionsantikörper und der Markersubstanz nötig. Unter Normalbedingungen erfolgt diese Durchmischung durch Zusammengeben der einzelnen Bestandteile und einer anschließenden Durchmischung mittels einer Bewegung von Reaktionsgefäßen, beispielsweise durch Rotationsmischer. Überschüssige Stoffmengen werden unter Normalbedingungen durch einfaches Ausschütten entfernt. Insbesondere unter Bedingungen reduzierter Schwerkraft, beispielsweise bei Experimenten im Weltraum, steht eine Entfernung überschüssiger Stoffmengen durch Schwerkraftwirkung nicht zur Verfügung. Zudem muss eine Durchmischung unter Bedingungen reduzierter Schwerkraft so erfolgen, dass andere, in der Nähe befindliche Experimente nicht gestört werden, beispielsweise aufgrund von Vibrationen.

Aus der WO 2005/069766 A2 ist bereits eine Vorrichtung zur Durchführung einer biochemischen Analyse bekannt, bei der zumindest ein Analyt in einer Probe selektiv bestimmt wird, mit zumindest einem Reaktionsbehälter, der zumindest ein Arbeitsvolumen aufweist, das zu einer Aufnahme eines Flüssigkeitsvolumens und zur Durchführung zumindest eines Teilschritts einer Analysereaktion vorgesehen ist, und mit zumindest einer Schnittstelle, die zu einer Verbindung zumindest eines Arbeitsvolumens mit einem weiteren Medienbehälter vorgesehen ist, wobei der Reaktionsbehälter als im montierten Zustand zumindest im Wesentlichen vollständig abgeschlossenes Gefäß ausgebildet ist und mit zumindest einem als Abfallbehälter ausgebildeten weiteren Medienbehälter, der zu einer Aufnahme überschüssiger Flüssigkeitsvolumina vorgesehen ist.

Ferner sind aus der WO 2010/017381 A2, der EP 0 144 162 A2, der US 2011/0071055 A1, der US 3,969,190 B, der US 2010/0311177 A1, der US 2002/095073 A1, der US 2007/0099288 A1, der US 2011/308313 A1, der US 2012/0003659 A1 und der US 2012/0149603 A1 ebenfalls bereits Vorrichtungen zur Durchführung einer biochemischen Analyse bekannt.

### Vorteile der Erfindung

Die Erfindung geht aus von einer Vorrichtung zur Durchführung einer biochemischen Analyse, insbesondere im Weltraum, insbesondere eines Immunoassays, bei der zumindest ein Analyt in einer Probe selektiv bestimmt wird, mit zumindest einem Reaktionsbehälter, der zumindest ein Arbeitsvolumen aufweist, das zu einer Aufnahme eines Flüssigkeitsvolumens und zur Durchführung zumindest eines Teilschritts einer Analysereaktion vorgesehen ist, und mit zumindest einer Schnittstelle, die zu einer Verbindung zumindest eines Arbeitsvolumens mit einem weiteren Medienbehälter vorgesehen ist. In dem Arbeitsvolumen können vor einem Beginn einer Analyse, insbesondere vor einer Zugabe der Probe, bereits Stoffe für eine Durchführung der Reaktion gebunden oder ungebunden gelagert sein. Grundsätzlich können anstelle von Flüssigkeitsvolumina auch Gasvolumina in dem Arbeitsvolumen aufgenommen werden, beispielsweise indem in einem Teilschritt einer Analyse ein Gas zu einer Verdrängung eines Flüssigkeitsvolumens eingeleitet wird. Unter "gebunden gelagert" soll insbesondere an eine Oberfläche des Arbeitsvolumens gebunden verstanden werden, wobei ein gebunden gelagerter Stoff in einem Verlauf eines Reaktionsprozesses abgelöst und in Lösung gebracht werden kann. Ferner soll unter "gebunden gelagert" verstanden werden, dass Stoffe an festen geometrischen Orten in dem Arbeitsvolumen irreversibel gebunden oder fixiert sind. Unter einer "Durchführung im Weltraum" soll insbesondere verstanden werden, dass die Durchführung der biochemischen Analyse außerhalb der Erde, beispielsweise in einem Raumfahrzeug in einem Erdorbit oder an einem Lagrange-Punkt, während eines Raumflugs oder eines Umlaufs um einen anderen Planeten oder um einen Mond, auf einem Satelliten, einem Mond, einem Asteroiden oder auf einem von der Erde verschiedenen Planeten erfolgt. Insbesondere kann die Durchführung im Weltall unter Bedingungen reduzierter Schwerkraft stattfinden. Unter "Bedingungen reduzierter Schwerkraft" sollen insbesondere Bedingungen verstanden werden, bei denen eine Schwerewirkung von maximal 0,9 g, vorteilhaft maximal 1*10⁻³ g, vorzugsweise maximal 1*10⁻⁶ g und besonders bevorzugt maximal 1*10⁻⁸ g wirksam ist. Die Schwerewirkung kann durch Gravitation und/oder künstlich durch eine Beschleunigung erzeugt sein. Mit "g" ist der Wert der Fallbeschleunigung auf der Erde von 9,81 m/s² bezeichnet. Unter einer "Schnittstelle" soll insbesondere ein Element verstanden werden, das dazu vorgesehen ist, eine vollständig geschlossene Verbindung zwischen dem Arbeitsvolumen und dem weiteren Medienbehälter herzustellen. Unter einer "vollständig geschlossenen Verbindung" soll insbesondere verstanden werden, dass ein Medienfluss über die Verbindung vollständig durch die Schnittstelle von einer äußeren Umwelt isoliert wird und insbesondere ein Stoffaustritt in die äußere Umwelt verhindert ist. Beispielsweise kann die Schnittstelle dazu ausgebildet sein, mit dem weiteren Medienbehälter eine Verbindung nach einem Luer-Lock-Prinzip auszubilden oder die Schnittstelle kann Septen aufweisen, wobei ein Stoffdurchlass durch die Septen mittels einer Penetration oder einem Verdrängen erreicht wird.

Ferner geht die Erfindung davon aus, dass der Reaktionsbehälter als im montierten Zustand zumindest im Wesentlichen vollständig abgeschlossenes Gefäß ausgebildet ist. Unter "zumindest im Wesentlichen vollständig abgeschlossen" soll insbesondere verstanden werden, dass das Gefäß wenigstens in einem zur Durchführung einer biochemischen Analyse montierten Zustand bis auf Ankopplungsöffnungen, die zu einer Ankopplung an weitere Gefäße zu einer Aufnahme von Reaktionsedukten oder Reaktionsprodukten vorgesehen sind, öffnungsfrei ausgebildet ist, so dass ein Austritt von Reaktionsedukten und/oder -produkten verhindert ist. Unter "im montierten Zustand zumindest im Wesentlichen vollständig abgeschlossen" soll insbesondere verstanden werden, dass der Reaktionsbehälter derart ausgebildet ist, dass eine Verbindung mit einem weiteren Element, beispielsweise einem kommerziell erhältlichen planaren Arrayträger für Fängerantikörper oder einer kommerziell erhältlichen Multiwell-Platte, zu einem vollständigen Abschluss des Reaktionsbehälters vorgesehen ist. Es kann insbesondere eine hohe Prozesssicherheit und eine universelle Einsetzbarkeit für eine Analyse von Gefahrstoffen, wie beispielsweise sauren, basischen oder toxischen Stoffen, und unter Extrembedingungen, wie beispielsweise Bedingungen reduzierter Schwerkraft, insbesondere im Weltraum, erreicht werden.

Des Weiteren geht die Erfindung davon aus, dass die Vorrichtung zumindest ein als Abfallbehälter, der zu einer Aufnahme überschüssiger Flüssigkeitsvolumina vorgesehen ist, ausgebildeter weiterer Medienbehälter aufweist. Unter "überschüssigen Flüssigkeitsvolumina" sollen insbesondere nach einem Teilschritt der biochemischen Analyse nicht mehr benötigte Flüssigkeitsvolumina verstanden werden, wie beispielsweise Probenvolumina mit ungebundenen Analyten nach Durchführung eines Teilschritts, in dem eine Bindung des Analyten an ortsfeste Fängerantikörper vorgesehen ist, oder Flüssigkeitsvolumina mit unreagierten Detektionsantikörpern. Vorzugsweise ist der Abfallbehälter über eine Verbindung mittels einer Schnittstelle zumindest mit einem Arbeitsvolumen verbunden. Es kann insbesondere ein kleines Arbeitsvolumen und eine kompakte Vorrichtung erreicht werden.

Es wird vorgeschlagen, dass der Abfallbehälter für einen Druckausgleichsbetrieb ein hydrophobes oder hydrophiles Filter für einen Druckausgleich mit einer Umgebung aufweist und dass der Abfallbehälter zumindest einen Dochtkörper aufweist, der mit Absorbermaterial gefüllt ist, das von tropffreiem organischem Schwammmaterial, kapillaren Kunststoffspeichern oder einem organischen Superabsorbermaterial, wie beispielsweise dem unter dem Handelsnamen Luquasorb® von der Firma BASF vertriebenen Produkt, gebildet ist.

Ferner wird vorgeschlagen, dass das Arbeitsvolumen für eine Reaktionsdurchführung unter Bedingungen reduzierter Schwerkraft ausgebildet ist. Insbesondere ist unter Bedingungen reduzierter Schwerkraft ein Verhalten von Flüssigkeiten durch eine Oberflächenspannung dominiert und das Arbeitsvolumen weist eine speziell an dieses Verhalten angepasste Gestaltung auf. Es kann insbesondere eine Vorrichtung erreicht werden, mit der eine Reaktion reproduzierbar und kontrolliert unter verminderten schwerkraftbedingten Störfaktoren durchgeführt werden kann.

Ferner wird vorgeschlagen, dass das Arbeitsvolumen eine sich von einer Schnittstelle aus auf-weitende Form aufweist. Unter einer "sich von einer Schnittstelle aus ausweitenden Form" soll insbesondere verstanden werden, dass das Arbeitsvolumen eine Form aufweist, bei der sich, in einer Ebene betrachtet, in der eine Längsausdehnung der Schnittstelle verläuft und in der ein Zustromvektor von Flüssigkeitsvolumina liegt, ausgehend von der Schnittstelle, ein Durchmesser des Arbeitsvolumens quer zu einer Ausströmungsrichtung aus der Schnittstelle bis zu einer Stelle maximaler Ausdehnung des Durchmessers des Arbeitsvolumens quer zu der Ausströmungsrichtung monoton vergrößert. Nach einer Stelle maximaler Ausdehnung kann der Durchmesser des Arbeitsvolumens quer zu der Ausströmungsrichtung sich in Ausströmungsrichtung insbesondere verringern. Insbesondere wird bei einer Einleitung von Flüssigkeitsvolumina in das Arbeitsvolumen somit eine rasche Vergrößerung einer von dem Flüssigkeitsvolumen bedeckten Oberfläche erreicht werden. Unter Bedingungen reduzierter Schwerkraft kann insbesondere bei Einleitung eines neuen Flüssigkeitsvolumens in ein von einem weiteren Flüssigkeitsvolumen zumindest teilweise gefülltes Volumen eine Verdrängung des weiteren Flüssigkeitsvolumens durch das neue Flüssigkeitsvolumen erreicht werden, da eine diffusive Mischung unter reduzierter Schwerkraft gering ist und aufgrund der Form des Arbeitsvolumens bei Einleitung des neuen Flüssigkeitsvolumens keine Restvolumina des weiteren Flüssigkeitsvolumens hinter einer Front des neuen Flüssigkeitsvolumens zurückbleiben. Es kann insbesondere ein Arbeitsvolumen erreicht werden, bei dem, insbesondere unter Bedingungen reduzierter Schwerkraft, ein Wechsel von Flüssigkeitsvolumina unter geringen Mischungsverlusten und/oder unter einer Minimierung eines benötigten zufließenden Volumens bei dem Wechsel von Flüssigkeitsvolumina in dem Arbeitsvolumen erreicht wird.

Ferner wird vorgeschlagen, dass das Arbeitsvolumen zumindest im Wesentlichen rechteckförmig ausgebildet ist. Unter einer "zumindest im Wesentlichen rechteckförmig" soll insbesondere verstanden werden, dass das Arbeitsvolumen, in zumindest einer Ebene betrachtet, vorzugsweise in einer Ebene, in der ein Zustromvektor von Flüssigkeitsvolumina liegt, eine rechteckige, vorzugsweise quadratische Form aufweist, wobei ein oder mehrere Ecken des Arbeitsvolumens abgerundet ausgebildet sein können. Es kann insbesondere ein Arbeitsvolumen erreicht werden, bei dem, insbesondere unter Bedingungen reduzierter Schwerkraft, ein Wechsel von Flüssigkeitsvolumina unter geringen Mischungsverlusten und/oder unter einer Minimierung eines benötigten zufließendem Volumens bei dem Wechsel von Flüssigkeitsvolumina in dem Arbeitsvolumen erreicht wird.

Ferner wird vorgeschlagen, dass das Arbeitsvolumen in einer Tropfen-Form ausgebildet ist. Unter einer Tropfen-Form soll insbesondere eine Form mit zumindest einer Eingangsöffnung und zumindest einer Ausgangsöffnung verstanden werden, bei der sich das Arbeitsvolumen in zumindest einer Ebene betrachtet, vorzugsweise in einer Ebene, in der ein Zustromvektor von Flüssigkeitsvolumina liegt, in zumindest einem Teilbereich auf die Ausgangsöffnung zu verbreitert. Es kann insbesondere ein Arbeitsvolumen erreicht werden, bei dem, insbesondere unter Bedingungen reduzierter Schwerkraft, ein Wechsel von Flüssigkeitsvolumina unter geringen Mischungsverlusten und/oder unter einer Minimierung eines benötigten zufließenden Volumens bei dem Wechsel von Flüssigkeitsvolumina in dem Arbeitsvolumen erreicht wird.

Ferner wird vorgeschlagen, dass das Arbeitsvolumen in einer Kreis-Form ausgebildet ist. Es kann insbesondere ein Arbeitsvolumen erreicht werden, bei dem, insbesondere unter Bedingungen reduzierter Schwerkraft, ein Wechsel von Flüssigkeitsvolumina unter geringen Mischungsverlusten und/oder unter einer Minimierung eines benötigten zufließenden Volumens bei dem Wechsel von Flüssigkeitsvolumina in dem Arbeitsvolumen erreicht wird.

Ferner wird vorgeschlagen, dass das Arbeitsvolumen in einer Düsen-Form ausgebildet ist. Unter einer "Düsen-Form" soll insbesondere eine Form mit zumindest einer Eingangsöffnung und zumindest einer Ausgangsöffnung verstanden werden, bei der sich das Arbeitsvolumen in zumindest einem Teilbereich auf die Ausgangsöffnung zu verjüngt. Vorzugsweise weist die Düsen-Form zumindest zwei Eingangsöffnungen auf. Es kann insbesondere ein Arbeitsvolumen erreicht werden, bei dem, insbesondere unter Bedingungen reduzierter Schwerkraft, ein Wechsel von Flüssigkeitsvolumina unter geringen Mischungsverlusten und/oder unter einer Minimierung eines benötigten zufließenden Volumens bei dem Wechsel von Flüssigkeitsvolumina in dem Arbeitsvolumen erreicht wird.

Ferner wird vorgeschlagen, dass der Abfallbehälter zumindest im Wesentlichen vollständig ab-geschlossen ausgebildet ist. Es kann insbesondere eine hohe Prozesssicherheit und eine universelle Einsetzbarkeit der Vorrichtung für eine Analyse von Proben, die Gefahrstoffe wie beispielsweise saure, basische oder toxische Stoffe enthalten, und/oder für eine unter Extrembedingungen stattfindende Analyse erreicht werden.

Ferner wird vorgeschlagen, dass der Abfallbehälter für einen Druckausgleichsbetrieb ausgebildet ist. Unter einem "Druckausgleichsbetrieb" soll insbesondere verstanden werden, dass der Abfallbehälter zumindest ein Filter zu einem Druckausgleich mit einer Umgebung aufweist, so dass bei einem Einleiten überschüssiger Flüssigkeitsvolumina, insbesondere unter Bedingungen reduzierter Schwerkraft, ein Druckaufbau innerhalb des Abfallbehälters und/oder eine Druckdifferenz zu einer Umgebung vermieden werden kann. In Abhängigkeit von verwendeten Medien in der Analyse ist das Filter als hydrophobes oder hydrophiles Filter ausgebildet. Es kann insbesondere ein Abfallbehälter mit einer erhöhten Sicherheit für einen Betrieb erreicht werden.

Ferner wird vorgeschlagen, dass der Abfallbehälter zumindest einen Dochtkörper aufweist. Unter einem "Dochtkörper" soll insbesondere ein kapillares Material verstanden werden, das dazu vorgesehen ist, beginnend von einem Einlass den Abfallbehälter zumindest teilweise an inneren Wandungen auszukleiden und einströmende Flüssigkeitsvolumina aufzunehmen und/oder weiterzuleiten. Der Dochtkörper kann insbesondere dazu vorgesehen sein, überschüssige Flüssigkeitsvolumina aufzunehmen und/oder ein Absorbermaterial zu lagern. Unter einem "Absorbermaterial" soll insbesondere ein Material verstanden werden, das dazu vorgesehen ist, Flüssigkeitsvolumina aufzunehmen und zu binden, beispielsweise organische Absorber, mineralische Adsorber, gesinterte Kunststoffspeicher, Aktivkohle oder Silikagel. Insbesondere ist der Dochtkörper dazu vorgesehen, in den Abfallbehälter eintretende überschüssige Flüssigkeitsvolumina aufzunehmen und zu einer verbesserten und beschleunigten Aufnahme durch das Absorbermaterial zu verteilen sowie ein Wiederaustreten aufgenommener Flüssigkeitsvolumina vorzugsweise zu verhindern. Vorzugsweise ist der Dochtkörper ferner dazu vorgesehen, zu einer Erreichung eines drucklosen Betriebs in dem Absorbermaterial enthaltenes Gas bei einer Aufnahme überschüssiger Flüssigkeitsvolumina zu einem von dem Absorbermaterial freien Bereich des Abfallbehälters zu leiten, von dem aus das Gas mittels eines Filters zu einem Druckausgleich abgegeben werden kann. Es kann insbesondere ein Abfallbehälter mit einer schnellen und sicheren Aufnahme überschüssiger Flüssigkeitsvolumina sowie einer Lagerung der überschüssigen Flüssigkeitsvolumina mit einer hohen Sicherheit erreicht werden.

Ferner wird zumindest ein als Analysematerialbehälter, der zu einer Bereitstellung von Analysematerialien vorgesehen ist, ausgebildeter weiterer Medienbehälter vorgeschlagen. Unter "Analysematerialien" sollen insbesondere für die Analysereaktion notwendige Materialien wie beispielsweise Fänger- und Markierungsantikörper und Markersubstanzen eines Immunoassays, die zu einer selektiven Bestimmung des Analyten dienen, sowie Hilfsstoffe wie Lösungsmittel und dergleichen verstanden werden. Vorzugsweise sind vor Beginn eines Verfahrens die Analyse-materialien in dem Analysematerialbehälter in einer benötigten volumetrischen Menge gelagert, sodass zu einer Durchführung des Verfahrens lediglich eine Abgabe der Analysematerialien erfolgen muss. Alternativ kann auch die Probe in dem Analysematerialbehälter gelagert sein und auf einen separaten Probenbehälter verzichtet werden. Es kann insbesondere eine operationell und volumetrisch sichere Zugabe der Analysematerialien erreicht werden.

Ferner wird vorgeschlagen, dass der Analysematerialbehälter als Mehrkammerspritze aus-gebildet ist. Unter einer "Mehrkammerspritze" soll insbesondere ein Behälter mit mehreren durch Trennelemente abgeteilten Teilkammern zu einer getrennten Lagerung verschiedener Reaktionsmaterialien verstanden werden. Vorzugsweise ist die Mehrkammerspritze dazu ausgebildet, die verschiedenen Analysematerialien sequentiell nacheinander abzugeben, wobei innerhalb der Mehrkammerspritze eine kontrollierte Mischung getrennt gelagerter Stoffe zu einer Stoffmischung vor einer Abgabe erfolgen kann. Vorzugsweise sind in der Mehrkammerspritze die benötigten Analysematerialien in einer speziell auf die Analyse zugeschnittenen Stoffmenge gelagert. Grundsätzlich kann anstatt einer Verwendung einer Mehrkammerspritze eine Lagerung von Komponenten der Analysematerialien jeweils in einem separaten Analysematerialbehälter vorgenommen werden. Es kann insbesondere eine Anzahl von Analysematerialienbehältern reduziert und Fehldurchführungen einer Analyse aufgrund von fehlenden und/oder in unzureichender Menge zugegebenen Analysematerialien vermieden werden.

Ferner wird vorgeschlagen, dass der Analysematerialbehälter integriert mit einem Abfallbehälter ausgeführt ist. Unter "integriert ausgeführt ist" soll insbesondere verstanden werden, dass der Analysematerialbehälter zumindest ein Kompartiment aufweist, das zu einer Aufnahme überschüssiger Flüssigkeitsvolumina vorgesehen ist und das vorzugsweise dazu vorgesehen ist, im Verlauf einer Analysereaktion parallel zu einer Entleerung von Analysematerial lagernden Kompartimenten überschüssige Flüssigkeitsvolumina aufzunehmen und während der Aufnahme ein Aufnahmevolumen dieses Kompartiments zu vergrößern. Das Kompartiment kann beispielsweise als Kammer des Analysematerialbehälters mit festem oder bevorzugt mit veränderbarem Aufnahmevolumen, beispielsweise als elastischer Aufnahmesack oder als Kammer, die mit einem verschiebbaren Element abgeschlossen ist, ausgebildet sein. Es kann insbesondere ein Verzicht auf einen zusätzlichen, separaten Abfallbehälter und eine Verringerung vorzuhaltenden Systemvolumens erreicht werden.

Ferner wird vorgeschlagen, dass zumindest ein Reaktionsbehälter zusammen mit zumindest einem weiteren Reaktionsbehälter und/oder zumindest einem weiteren Medienbehälter zu einem Modul, das zu einer Verbindung mit einem weiteren Medienbehälter vorgesehen ist, vormontiert ist. Vorzugsweise weist das Modul neben dem Reaktionsbehälter einen Abfallbehälter und einen Analysematerialbehälter auf, sodass zu einer Durchführung der biochemischen Analyse lediglich der Probenbehälter über eine Schnittstelle angeschlossen werden muss. Vorzugsweise sind der Reaktionsbehälter und der Analysematerialbehälter bereits mit Analysematerialien befüllt, sodass auf einen Befüllungsschritt vor Durchführung einer biochemischen Analyse verzichtet werden kann. Es kann insbesondere eine Zeitersparnis in der Durchführung der biochemischen Analyse durch eine Vormontage von Arbeitseinheiten erreicht werden.

Ferner wird vorgeschlagen, dass das Modul dazu vorgesehen ist, eine parallele Durchführung mehrerer biochemischer Analysen zu ermöglichen. Insbesondere weist das Modul hierzu mehrere Reaktionsbehälter und/oder einen Reaktionsbehälter mit mehreren Arbeitsvolumina auf. Insbesondere weist das Modul hierzu eine Konfiguration auf, in der mehrere Reaktionsbehälter und/oder Arbeitsvolumina parallel angeordnet sind. Es kann insbesondere eine Zeit- und Platzersparnis erreicht werden.

Ferner werden magnetische Mischungskörper, die zu einer Durchmischung von Reaktionsmaterialien und der Probe für die Analysereaktion vorgesehen sind, vorgeschlagen. Unter "magnetischen Mischungskörpern" sollen insbesondere magnetische und/oder magnetisierbare Körper verstanden werden, die dazu vorgesehen sind, mittels eines angelegten Magnetfelds, vorzugsweise eines angelegten magnetischen Wechselfelds, zu einer Durchmischung der Reaktionsmaterialien bewegt zu werden. Es kann insbesondere eine sichere Durchmischung der Reaktionsmaterialien erreicht werden.

Ferner wird ein Verfahren zur Durchführung einer biochemischen Analyse mit einer erfindungs-gemäßen Vorrichtung vorgeschlagen, bei dem die Durchführung unter Bedingungen reduzierter Schwerkraft erfolgt. Insbesondere ist das Verfahren derart ausgebildet, dass sämtliche Teilschritte der Durchführung unabhängig von einem Vorhandensein einer Schwerkraft durchgeführt werden können. Es kann insbesondere eine Vermeidung von schwerkraftbedingten oder mechanisch verursachten Störeinflüssen erreicht werden.

Ferner wird vorgeschlagen, dass eine Durchmischung von Analysematerialien und der Probe für die Analysereaktion mittels magnetischer Mischungskörper erfolgt. Es kann insbesondere eine vollständige und effiziente Durchmischung, insbesondere unter Bedingungen reduzierter Schwerkraft im Weltraum, erreicht werden.

Ferner wird vorgeschlagen, dass an einer Analysereaktion nur Analysematerialien und Proben innerhalb eines Arbeitsvolumens eines Reaktionsbehälters beteiligt sind. Insbesondere kann auf eine volumetrisch hochgenaue Bereitstellung von benötigten Volumina an Analysematerialien und/oder Proben verzichtet werden und stattdessen bis zu einer Füllung des Arbeitsvolumens, durch das ein Volumen beteiligter Stoffe vorgegeben ist, Analysematerialien und/oder Proben hinzugegeben werden. Es kann insbesondere ein einfach durchführbares und besonders unter Bedingungen reduzierter Schwerkraft einfach durchführbares Verfahren erreicht werden.

Ferner wird vorgeschlagen, dass eine Zugabe von Analysematerialien und Proben in beliebig kleinen Subvolumina und mit eingelegten Pausen ablaufen kann. Es kann insbesondere ein flexibel anpassbares Verfahren erreicht werden.

Die erfindungsgemäße Vorrichtung soll hierbei nicht auf die oben beschriebene Anwendung und Ausführungsform beschränkt sein. Insbesondere kann die erfindungsgemäße Vorrichtung zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind vierundzwanzig Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer erfindungsgemäßen Vorrichtung mit zwei Reaktionsbehältern mit jeweils einem Arbeitsvolumen, einem Analysematerialienbehälter, einem Abfallbehälter und einem Probenbehälter,
- Fig. 2: eine Detailansicht eines erfindungsgemäßen Reaktionsbehälters,
- Fig. 3: eine schematische Darstellung eines Arbeitsvolumens in einer Kreis-
Form,
- Fig. 4: eine schematische Darstellung eines alternativen Arbeitsvolumens in einer Rechteck-Form,
- Fig. 5: eine schematische Darstellung eines alternativen Arbeitsvolumens in einer Tropfen-Form,
- Fig. 6: eine schematische Darstellung eines alternativen Arbeitsvolumens in einer Düsen-Form, das zwei Einlässe und einen Auslass aufweist,
- Fig. 7: eine schematische Darstellung eines Prozessablaufs einer biochemischen Analyse in einer erfindungsgemäßen Vorrichtung,
- Fig. 8: Einzelteile eines Reaktionsbehälters aus Fig. 2 vor einem Zusammenbau in einer Seitenansicht,
- Fig. 9: den Reaktionsbehälter aus Fig. 2 in einem teilweise zusammengebauten Zustand in einer schematischen Seitenansicht,
- Fig. 10: den Reaktionsbehälter aus Fig. 2 in einem zusammengebauten Zustand in einer schematischen Seitenansicht,
- Fig. 11: eine alternative Ausführung eines Reaktionsbehälters mit einem variierbaren Arbeitsvolumen in einer schematischen Seitenansicht,
- Fig. 12: eine alternative Ausführung eines Reaktionsbehälters mit einem variierbaren Arbeitsvolumen in einer schematischen Seitenansicht,
- Fig. 13: eine Ausführung eines Reaktionsbehälters mit einer alternativen Schnittstellenanordnung in einer schematischen Seitenansicht,
- Fig. 14: eine Ausführung eines Reaktionsbehälters mit einer alternativen Schnittstellenanordnung in einer schematischen Seitenansicht,
- Fig. 15: eine Ausführung eines Reaktionsbehälters mit einer alternativen Schnittstellenanordnung in einer schematischen Seitenansicht,
- Fig. 16: eine Ausführung eines Reaktionsbehälters mit einer alternativen Schnittstellenanordnung in einer schematischen Seitenansicht,
- Fig. 17: eine alternative Bereitstellung von Fängerantikörpern in einem Reaktionsbehälter nach Fig. 2, gebunden an Magnetträgerkörper und in einer Lösung,
- Fig. 18: eine alternative Bereitstellung von Fängerantikörpern in einem Reaktionsbehälter nach Fig. 2, die auf einem separaten Trägermaterial fixiert oder getrocknet vorliegen,
- Fig. 19: eine alternative Bereitstellung von Fängerantikörpern in einem Reaktionsbehälter nach Fig. 2 als eingetrocknete Dots, die in einer Reaktionsdurchführung abgelöst werden,
- Fig. 20: eine schematische Darstellung eines Abfallbehälters aus Fig. 1 bei einem Füllvorgang,
- Fig. 21: eine schematische Darstellung eines alternativen Abfallbehälters bei einem Füllvorgang,
- Fig. 22: eine schematische Darstellung eines alternativen Abfallbehälters vor einem Füllvorgang,
- Fig. 23: eine schematische Darstellung eines alternativen Abfallbehälters bei einem Füllvorgang,
- Fig. 24: eine schematische Darstellung eines alternativen Abfallbehälters,
- Fig. 25: eine alternative Vorrichtung mit einem Reaktionsbehälter, der zwei Arbeitsvolumina aufweist,
- Fig. 26: eine alternative Vorrichtung, bei der Reaktionsbehälter, Analysematerialienbehälter und Abfallbehälter zu einem Modul vormontiert sind,
- Fig. 27: eine alternative Vorrichtung zu einer parallelen Durchführung mehrerer biochemischer Analysen,
- Fig. 28: eine alternative Vorrichtung zu einer parallelen Durchführung mehrerer biochemischer Analysen, bei der mehrere Reaktionsbehälter zu einem Modul vormontiert sind,
- Fig. 29: eine alternative Vorrichtung, bei der ein Modul aus mehreren Reaktions-behältern sukzessive durch Überdruck von einem Multiportventil beschickt wird,
- Fig. 30: eine alternative Vorrichtung, bei der ein Modul aus mehreren Reaktions-behältern sukzessive durch Unterdruck von einem Multiportventil beschickt wird,
- Fig. 31: eine alternative Vorrichtung zu einer parallelen Durchführung mehrerer biochemischer Analysen, bei der mehrere Reaktionsbehälter zu einem Modul vormontiert sind,
- Fig. 32: eine alternative Vorrichtung, bei der der Reaktionsbehälter durch eine Verbindung mit einer kommerziellen Multiwell-Platte vollständig abgeschlossen wird,
- Fig. 33: eine alternative Vorrichtung, bei der der Reaktionsbehälter durch eine Verbindung mit einem kommerziellen planaren Array vollständig abgeschlossen wird und
- Fig. 34: eine alternative Vorrichtung mit einem Analysematerialbehälter, der integriert mit einem Abfallbehälter ausgeführt ist.

### Beschreibung der Ausführungsbeispiele

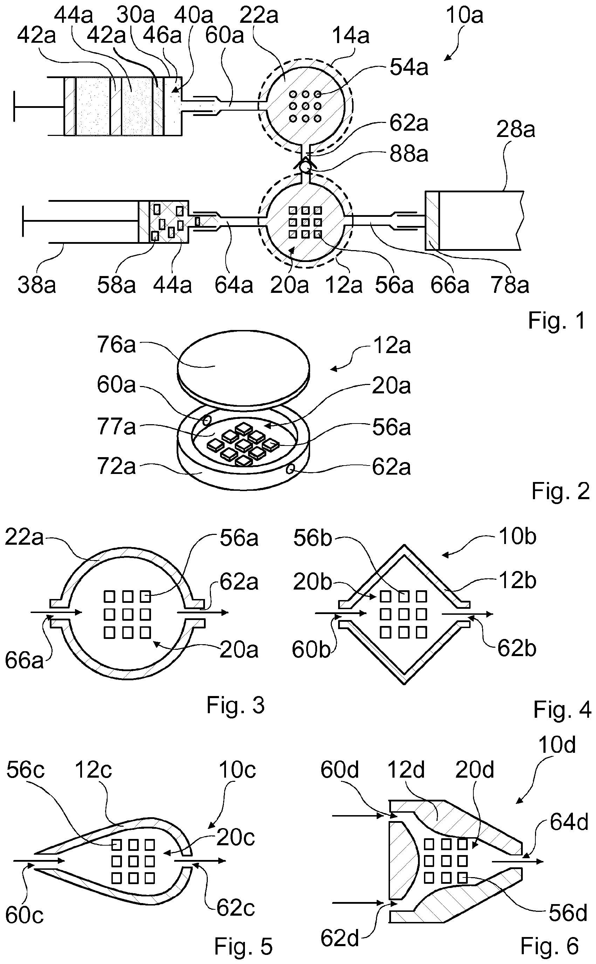

Fig. 1 zeigt in einer Draufsicht eine erfindungsgemäße Vorrichtung 10a zur Durchführung einer von einem Immunoassay gebildeten biochemischen Analyse im Weltraum, bei der ein Analyt in einer Probe 44a selektiv bestimmt wird, mit zwei Reaktionsbehältern 12a, 14a, die jeweils ein Arbeitsvolumen 20a, 22a aufweisen, die zu einer Aufnahme eines Flüssigkeitsvolumens und zur Durchführung zumindest eines Teilschritts einer Analysereaktion vorgesehen sind, und mit vier Schnittstellen 60a, 62a, 64a, 66a, die zu einer Verbindung zumindest der zwei Arbeitsvolumina 20a, 22a untereinander und mit drei weiteren Medienbehälter 28a, 30a, 38a vorgesehen sind. Die Schnittstelle 62a zwischen den Arbeitsvolumina 20a, 22a weist ein Ventil 88a zu einer Verhinderung eines Rückflusses von dem Arbeitsvolumen 20a in das Arbeitsvolumen 22a auf. Die Reaktionsbehälter 12a, 14a sind als im montierten Zustand im Wesentlichen vollständig abgeschlossene Gefäße ausgebildet, die lediglich über die Schnittstellen 60a, 62a, 64a, 66a zugänglich sind. Die Arbeitsvolumina 20a, 22a sind für eine Reaktionsdurchführung unter Bedingungen reduzierter Schwerkraft ausgebildet und weisen eine an das unter Bedingungen reduzierter Schwerkraft durch eine Oberflächenspannung dominierte Verhalten von Flüssigkeiten speziell an dieses Verhalten angepasste Gestaltung auf. Die Arbeitsvolumina 20a, 22a weisen eine sich von einer Schnittstelle 60a, 62a bzw. 62, 64a, 66a aus aufweitende Form auf. Die Arbeitsvolumina 20a, 22a sind in einer Kreis-Form ausgebildet. In dem Arbeitsvolumen 20a des Reaktionsbehälters 12a sind Fängerantikörper 56a für den Immunoassay bereits vor einem Beginn des Immunoassays an eine Oberfläche des Arbeitsvolumens 20a gebunden und in dem Arbeitsvolumen 22a des Reaktionsbehälters 14a sind Detektionsantikörper 54a in einer getrockneten Form bereits vor dem Beginn des Immunoassays enthalten und werden in einem Verlauf des Immunoassays in Lösung gebracht.

Die Vorrichtung 10a umfasst ferner einen als Abfallbehälter 28a, der zu einer Aufnahme überschüssiger Flüssigkeitsvolumina vorgesehen ist, ausgebildeten weiteren Medienbehälter. Der Abfallbehälter 28a nimmt im Verlauf des Verfahrens nicht mehr benötigte Flüssigkeitsvolumina wie beispielsweise Probenüberreste mit unreagiertem und nicht an Fängerantikörper 56a gebundenem Analyten auf und ist mit dem Arbeitsvolumen 20a über eine Schnittstelle 66a verbunden. Der Abfallbehälter 28a weist einen Kolben 78a zu einer Vergrößerung eines Aufnahmevolumens auf und ist im Wesentlichen vollständig abgeschlossen ausgebildet. Die Vorrichtung 10a weist ferner einen als Probenbehälter 38a ausgebildeten weiteren Medienbehälter auf, der mit dem Arbeitsvolumen 20a über die Schnittstelle 64a verbunden ist und zu einer Zuführung der Probe 44a vorgesehen ist. In dem Probenbehälter 38a sind neben der Probe 44a magnetische Mischungskörper 58a gelagert, die zu einer Durchmischung von Analysematerialien 46a, 48a, 50a, 52a und der Probe 44a für eine Analysereaktion vorgesehen sind. Der Probenbehälter 38a ist im Wesentlichen vollständig abgeschlossen ausgebildet. Die Vorrichtung 10a weist ferner einen als Analysematerialbehälter 30a, der zu einer Bereitstellung von Analysematerialien 46a, 48a, 50a, 52a vorgesehen ist, ausgebildeten weiteren Medienbehälter auf, der im Wesentlichen vollständig abgeschlossen ausgebildet ist. Der Analysematerialbehälter 30a ist als Mehrkammerspritze mit mehreren durch Trennelemente 42a abgetrennte Teilkammern 40a, die zu einer getrennten Lagerung unterschiedlicher Analysematerialien 46a, 48a, 50a, 52a vorgesehen sind, ausgebildet. Eine Verbindung des Analysematerialienbehälters 30a mit dem Arbeitsvolumen 22a wird über die Schnittstelle 60a erreicht. Der Analysematerialbehälter 30a ist ferner zu einer sequentiellen Abgabe getrennt gelagerter Analysematerialien 46a, 48a, 50a, 52a ausgebildet. Bereits vor einem Transport der Vorrichtung 10a in den Weltraum ist der Analysematerialbehälter 30a mit den Analysematerialien46a, 48a, 50a, 52a befüllt worden, die für die Durchführung der biochemischen Analyse benötigt werden.

In Fig. 2 wird der Reaktionsbehälter 12a mit dem Arbeitsvolumen 20a in einer genaueren An-sicht in einem teilweise zusammengebauten Zustand dargestellt. Der Reaktionsbehälter 12a ist aus einem Grundkörper 72a, einem Boden 74a und einem darauf zu setzenden Deckel 76a zusammengesetzt. Ein Material des Reaktionsbehälters 12a ist von einem transparenten Cyclo-Olefin-Copolymer gebildet, welches eine geringe unspezifische Bindungsfähigkeit aufweist und eine Auswertung des Immunoassays mittels optischer Methoden aufgrund einer Transparenz erlaubt. Grundsätzlich kann der Reaktionsbehälter 12a auch aus einem anderen Material, beispielsweise aus sogenanntem "low-binding" Polystyrol, wobei das Material vorteilhaft von einem Kunststoff und vorzugsweise von einem transparenten Kunststoff gebildet ist.

Fig.3 zeigt eine schematische Darstellung des Arbeitsvolumens 20a, das in einer Kreis-Form mit gegenüberliegenden Schnittstellen 60a, 62a ausgebildet ist.

In den Figuren 4-34 sind, neben weiteren Details des ersten Ausführungsbeispiels der Erfindung, zweiundzwanzig weitere Ausführungsbeispiele der Erfindung dargestellt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der Fig. 1 verwiesen wird. Zur Unterscheidung der Ausführungsbeispiele ist der Buchstabe a den Bezugszeichen des ersten Ausführungsbeispiels in den Fig. 1-3 nachgestellt. In den weiteren Ausführungsbeispielen der Fig. 4 bis 33 ist der Buchstabe a durch die Buchstaben b bis w ersetzt. In den Fig. 4 bis 33 ist bei den weiteren Ausführungsbeispielen bei sich auf das erste Ausführungsbeispiel beziehende Figurenbeschreibungen der Buchstabe a beibehalten.

In Fig. 4 ist ein Ausschnitt einer alternativen Vorrichtung 10b mit einem Reaktionsbehälter 12b mit einem Arbeitsvolumen 20b, das rechteckförmig ausgebildet ist, dargestellt. In dem Arbeitsvolumen 20b sind Fängerantikörper 56b fest gebunden. In zwei gegenüberliegenden Ecken der Rechteck-Form sind Schnittstellen 60b, 62b angeordnet. Grundsätzlich können die Schnittstellen 60b, 62b auch an benachbarten Ecken der Rechteck-Form angeordnet sein oder zumindest eine der Schnittstellen 60b, 62b kann in einem Wandungsbereich zwischen Ecken, am Boden oder Deckel angeordnet sein. Das Arbeitsvolumen 20b weist ebenfalls eine sich von einer Schnittstelle 60b, 62b aus aufweitende Form auf. In alternativen Ausgestaltungen der Vorrichtung 10b können ein oder mehrere der Ecken des rechteckförmigen Arbeitsvolumens 20b, vorzugsweise von den Schnittstellen 60b, 62b entfernte Ecken, abgerundet ausgebildet sein.

Eine alternative Vorrichtung 10c weist einen Reaktionsbehälter 12c (Fig. 5) mit einem Arbeitsvolumen 20c auf, der in einer Tropfen-Form ausgebildet ist. An einer spitz zulaufenden Stelle eines Rands der Tropfen-Form ist eine Schnittstelle 60c angeordnet und an einer der spitz zulaufenden Stelle gegenüberliegenden Stelle eine Schnittstelle 62c. Ausgehend von der Schnittstelle 60c weitet sich das Arbeitsvolumen 20c auf, wodurch infolge einer Oberflächenspannung ein Haften einströmender Flüssigkeiten an Wandungen des Arbeitsvolumens und unter Bedingungen reduzierter Schwerkraft ein Wechsel von Flüssigkeitsvolumina unter geringen Mischungsverlusten und mit einem reduzierten Volumen eines nachfolgenden Mediums erreicht wird.

Eine alternative Vorrichtung 10d umfasst einen Reaktionsbehälter 12d (Fig. 6) mit einem Arbeitsvolumen 20d, das in einer Düsen-Form ausgebildet ist. Das Arbeitsvolumen 20d weist an einer Seite zwei Schnittstellen 60d, 62d sowie an einer den beiden Schnittstellen 60d, 62d gegenüberliegenden Seite eine Schnittstelle 64d auf. Über die zwei Schnittstellen 60d, 62d können zwei verschiedene Stoffzuflüsse gleichzeitig oder aufeinanderfolgend bereitgestellt werden, wodurch eine Prozessdauer verkürzt und Stoffverluste durch Wechseln eines Medienbehälters vermieden werden können.

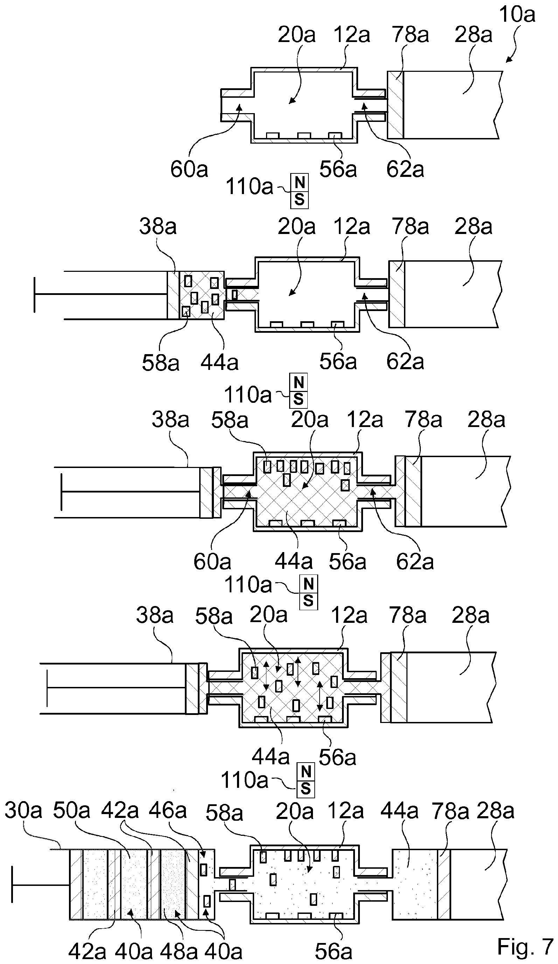

Fig. 7 zeigt eine beispielhafte Darstellung eines Verfahrens zur Durchführung einer biochemischen Analyse in der Vorrichtung 10a. Die Durchführung erfolgt unter Bedingungen reduzierter Schwerkraft an Bord eines Raumfahrzeugs im Weltall. Grundsätzlich kann die Durchführung des Verfahrens auch auf einem Asteroiden, einem Mond oder sogar auch auf der Erde erfolgen. Eine Durchmischung von Analysematerialien 46a, 48a, 50a, 52a und der Probe 44a für die Analysereaktion erfolgt mittels magnetischer Mischungskörper 58a. In einem ersten Verfahrensschritt ist der Reaktionsbehälter 12a lediglich mit den gebundenen Fängerantikörpern 56a befüllt und mit dem Abfallbehälter 28a über die Schnittstelle 62a verbunden. In einem weiteren Verfahrensschritt wird der Probenbehälter 38a über die Schnittstelle 60a mit dem Arbeitsvolumen 20a verbunden. In einem folgenden Verfahrensschritt wird Druck auf einen verschiebbaren Kolben in dem Probenbehälter 38a ausgeübt und durch den Druck Material der Probe 44a mit den magnetischen Mischungskörpern 58a in das Arbeitsvolumen 20a verschoben. In einem folgenden Verfahrensschritt wird eine Durchmischung der Probe 44a mittels der magnetischen Mischungskörper 58a bewirkt, die über eine Magneteinheit 110a in Bewegung versetzt werden, wodurch ein in der Probe 44a enthaltener Analyt an Positionen der Fängerantikörper 56a vorbeigeführt wird und an diese bindet. Bei einem Befüllen wird überschüssiges Volumen der Probe 44a über die Schnittstelle 62a von dem Arbeitsvolumen 20a des Reaktionsbehälters 12a in den Abfallbehälter 28a verschoben. In einem folgenden Reaktionsschritt wird der Probenbehälter 38a gegen den Analysematerialbehälter 30a ausgetauscht. Über Druck auf einen beweglichen Kolben des Analysematerialbehälters 30a wird analog zu einer Entleerung des Probenbehälters 38a der Analysematerialbehälter 30a entleert und Analysematerialien 46a, 48a, 50a, 52a sukzessive in das Arbeitsvolumen 20a eingeführt. Beispielsweise kann das Analysematerial 46a von einer neutralen Spüllösung gebildet sein, mittels derer Flüssigkeitsvolumen mit ungebundenem Analyten aus dem Arbeitsvolumen 20a verdrängt und in den Abfallbehälter 28a verschoben wird. Das Analysematerial 48a kann dann beispielsweise von einer Lösung mit Detektionsantikörpern 54a mit daran gebundenem Markermaterial, das als Enzym zu einer Spaltung eines Substrats zu einer Signalgebung ausgebildet ist, gebildet sein. Das Analysematerial 48a wird mittels der Magneteinheit 110a und der magnetischen Mischungskörper 58a durchmischt und Detektionsantikörper 54a mit Markern binden an den an die Fängerantikörper 56a gebundenen Analyten. In einem weiteren, beispielhaften Schritt werden mit einer weiteren Spüllösung 52 ungebundene Detektionsantikörper 54a aus dem Arbeitsvolumen entfernt. In einem eigentlichen Nachweisschritt des beispielhaften Verfahrens wird zu Erzeugung eines Nachweissignals mit dem Analysematerial 52a Substrat zur Spaltung durch die Marker eingeführt, das nach Spaltung beispielsweise ein Fluoreszenzsignal erzeugt. Überschüssige Flüssigkeitsvolumina werden während des Verfahrens in den Abfallbehälter 28a überführt. Bei dem Verfahren sind an einer Analysereaktion nur Analysematerialien 46a, 48a, 50a, 52a und die Probe 44a innerhalb des Arbeitsvolumens 20a des Reaktionsbehälters 12a beteiligt, wodurch auf ein volumetrisch hochgenaues Abmessen von Analysematerialien 46a, 48a, 50a, 52a und der Probe 44a verzichtet werden kann. Im Zuge eines Ablaufs des Verfahrens kann eine Zugabe von Analysematerialien 46a, 48a, 50a, 52a und der Probe 44a in beliebig kleinen Subvolumina und mit eingelegten Pausen ablaufen. In alternativen Verfahrensabläufen können die Fängerantikörper 56a beispielsweise an magnetischen Trägerkörpern gebunden zugegeben werden anstatt an dem Boden 74a gebunden zu sein. Desweiteren können in alternativen Verfahrensabläufen für jedes der Analysematerialien 46a, 48a, 50a, 52a separate Einzelmaterialienbehälter anstatt der als Mehrkammerspritze ausgebildete Analysematerialienbehälter 30a verwendet werden. Grundsätzlich können anstelle von Immunoassays auch andere biochemische Analyseverfahren in der Vorrichtung 10a durchgeführt werden. Die einzelnen Teilschritte des Verfahrens sind in einer Durchführung unabhängig von einem Vorhandensein einer Schwerkraft und können somit unter Bedingungen reduzierter Schwerkraft durchgeführt werden. Grundsätzlich ist jedoch auch eine Durchführung unter normalen Schwerkraftbedingungen auf der Erde möglich.

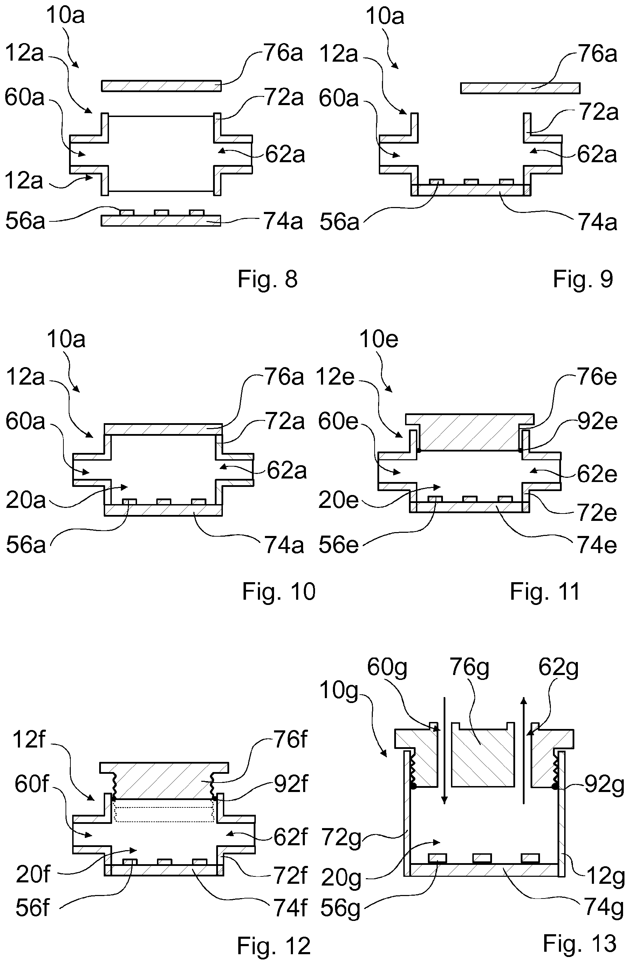

In Fig. 8 - 10 ist ein Montagevorgang des Reaktionsbehälters 12a dargestellt. In einem Montageschritt (Fig. 8) ist der Reaktionsbehälter 12a in von dem Deckel 76a, dem Grundkörper 72a mit den Schnittstellen 60a und 62a und dem Boden 74a mit daran gebundenen Fängerantikörpern 56a gebildeten Einzelteilen zerlegt. In einem folgenden Montageschritt (Fig. 9) wird der Boden 74a in den Grundkörper 72a eingesetzt und mittels eines Klebevorgangs fest befestigt. In einem letzten Montageschritt (Fig. 10) wird der Deckel 76a aufgesetzt und ebenfalls mit einem Klebevorgang befestigt. Alternativ kann anstatt eines Klebevorgangs auch ein anderer Befestigungsvorgang, beispielsweise ein Schweißprozess oder eine kraft- und/oder formschlüssige Befestigung des Deckels 76a in dem Grundkörper 72a, vorgenommen werden.

In Fig. 11 ist ein alternativer Reaktionsbehälter 12e einer alternativen Vorrichtung 10e dargestellt, der einen Grundkörper 72e mit Schnittstellen 60e, 62e an Seitenbereichen und einen Boden 74e mit daran gebundenen Fängerantikörpern 56e umfasst. Ein Deckel 76e ist in den Grundkörper 72e eingepresst und mit einem O-Ring 92e abgedichtet. Grundkörper 72e, Boden 74e und Deckel 76e begrenzen ein kreisförmiges Arbeitsvolumen 20e. Über eine Variation einer Einpresstiefe des Deckels 76e ist eine Einstellung des Arbeitsvolumens 20e möglich.

Eine weitere alternative Vorrichtung 10f (Fig. 12) weist einen Reaktionsbehälter 12f mit einem in einen Grundkörper 72f mit seitlichen Schnittstellen 60f, 62f einschraubbaren Deckel 76f auf. Der Deckel 76f kann in verschiedenen Tiefen eingeschraubt werden, worüber ein Arbeitsvolumen 20f des Reaktionsbehälters variiert werden kann, und wird mit einem O-Ring 92f abgedichtet. An einem Boden 74f sind Fängerantikörper 56f gebunden.

Fig. 13 zeigt einen alternativen Reaktionsbehälter 12g einer alternativen Vorrichtung 10g, der im Wesentlichen analog zu dem vorherigen Ausführungsbeispiel ausgebildet ist, mit einem Arbeitsvolumen 20g. Ein in einen Grundkörper 72g eingeschraubter Deckel 76g ist mit einem O-Ring 92g abgedichtet und weist zwei Schnittstellen 60g, 62g auf, die zu einer Zu- und Ableitung von Flüssigkeitsvolumina vorgesehen sind. An einem Boden 74g sind Fängerantikörper 56g für eine Durchführung eines Immunoassays gebunden. Alternativ können für die Durchführung eines Immunoassays auch Detektionsantikörper oder andere benötigte Analysematerialien an dem Boden 74g gebunden sein

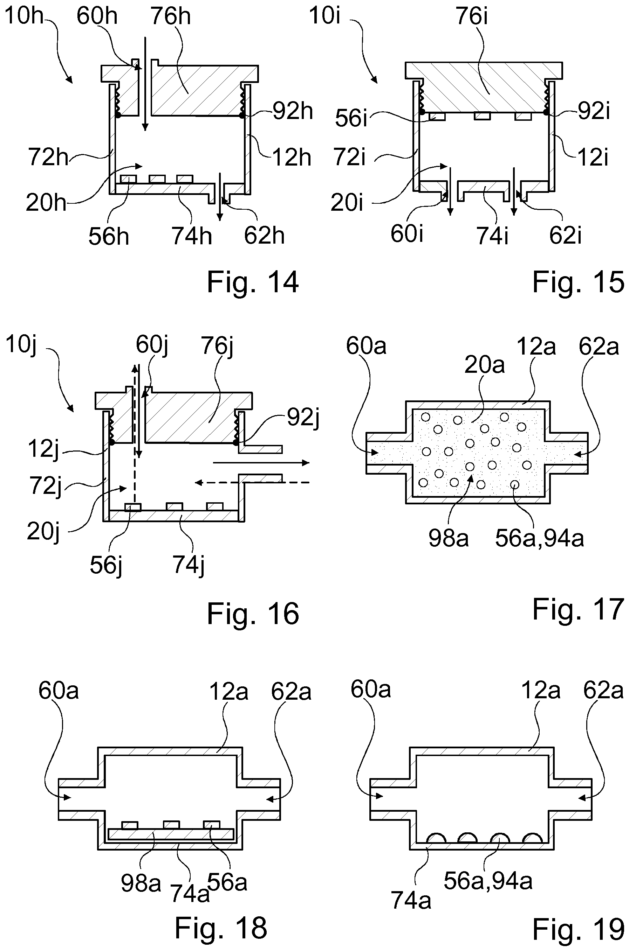

Ein weiterer alternativer Reaktionsbehälter 12h (Fig. 14) einer alternativen Vorrichtung 10h weist einen in einen Grundkörper 72h eingeschraubten Deckel 76h auf, der eine Schnittstelle 60h zu einer Flüssigkeitseinleitung in ein Arbeitsvolumen 20h aufweist und mit einem O-Ring 92h abgedichtet ist. An einem Boden 74h sind Fängerantikörper 56h gebunden und eine Schnittstelle 62h zu einer Flüssigkeitsableitung angeordnet. Unter Bedingungen reduzierter Schwerkraft können Ein- und Ableitung von Flüssigkeiten in beliebigen Richtungen erfolgen.
In einer weiteren alternativen Vorrichtung 10i (Fig. 15) weist ein Reaktionsbehälter 12i, der ein Arbeitsvolumen 20i aufweist, mit einem Deckel 76i, der in einen Grundkörper 72i eingeschraubt und mit einem O-Ring 92i abgedichtet ist, auf. An dem Deckel 76i sind Fängerantikörper 56i gebunden angeordnet. Alternativ können anstatt Fängerantikörpern 56i auch Detektionsantikörper oder andere benötigte Analysematerialien an dem Deckel 76i gebunden angeordnet sein. Schnittstellen 60i, 62i sind an einem Boden 74i angeordnet.

In einem weiteren Reaktionsbehälter 12j (Fig. 16) mit einer alternativen Anordnung von Schnittstellen 60j, 62j ist die Schnittstelle 60j zu einer Einleitung von Flüssigkeitsvolumina in ein Arbeitsvolumen 20j in einem Deckel 76j, der in einen Grundkörper 72j eingeschraubt und mit einem O-Ring 92j abgedichtet ist, angeordnet. An einem Boden 74j sind Fängerantikörper 56j gebunden angeordnet. Alternativ können anstatt Fängerantikörpern 56j auch Detektionsantikörper oder andere benötigte Analysematerialien an dem Boden 74j gebunden angeordnet sein. Die Schnittstelle 62j, die zu einer Ableitung von Flüssigkeitsvolumina vorgesehen ist, ist an einem Seitenbereich des Grundkörpers 72j angeordnet.

In Fig. 17 ist eine alternative Lagerung der Fängerantikörper 56a in dem Reaktionsbehälter 12a dargestellt. Die Fängerantikörper 56a sind an Magnetträgerkörpern 94a gebunden angeordnet und sind mit diesen in einer Suspension 98a in dem Arbeitsvolumen 20a freischwebend angeordnet. Die Magnetträgerkörper 94a sind dazu vorgesehen, von der Magneteinheit 110a bewegt zu werden. Alternativ können anstatt Fängerantikörpern 56a auch Detektionsantikörper 54a oder andere benötigte Analysematerialien an den Magnetträgerkörpern 94a gebunden angeordnet sein

Bei einer weiteren alternativen Lagerung (Fig. 18) der Fängerantikörper 56a sind diese gebunden auf einem Träger 96a aus Kunststoff oder Glas oder einem anderen transparenten Material angeordnet, der auf dem Boden 74a ausgelegt ist. Alternativ kann der Träger 96a jedoch auch auf dem Boden 74a angeschweißt oder angeklebt oder an einem Deckel 76a befestigt sein. In einer weiteren alternativen Ausgestaltung können anstatt Fängerantikörper 56a auf die oben angegebene Weise auch Detektionsantikörper 54a gelagert werden.

Bei einer weiteren alternativen Lagerung (Fig. 19) der Fängerantikörper 56a sind diese gebunden an Magnetträgerkörper 94a an einem Boden 74a des Reaktionsbehälters 12a in einem getrockneten Zustand angeordnet. Durch Zuleitung eines Flüssigkeitsvolumens durch eine der Schnittstellen 60a, 62a werden diese in Lösung gebracht und sind dann zu einer Durchmischung mittels der Magneteinheit 110a vorgesehen. In einer weiteren alternativen Ausgestaltung können anstatt oder zusätzlich zu den Fängerantikörpern 56a auf die oben angegebene Weise auch Detektionsantikörper 54a gelagert werden. Ebenso ist es alternativ möglich, Fängerantikörper 56a und Detektionsantikörper 54a gemischt an derselben Oberfläche zu lagern. In einer weiteren alternativen Ausgestaltung können Fängerantikörper 56a am Boden 74a oder am Deckel 76a des Reaktionsbehälters 12a und Detektionsantikörper 54a am Deckel 76a oder am Boden 74a des Reaktionsbehälters 12a, jeweils an Magnetträgerkörper 94a gebunden, in getrocknetem Zustand voneinander getrennt gelagert werden.

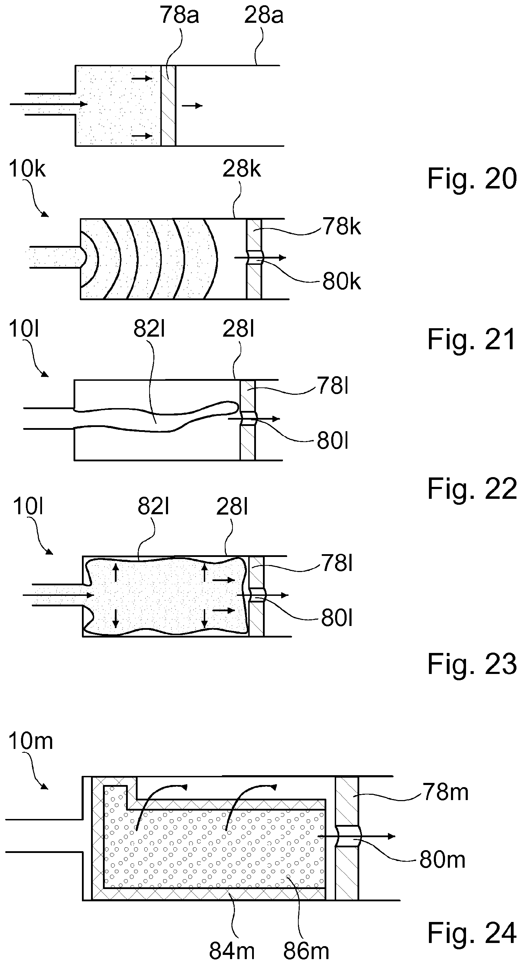

In Fig. 20 ist der Abfallbehälter 28a der Vorrichtung 10a bei einem Befüllungsvorgang dargestellt. Der Abfallbehälter 28a weist einen verschiebbaren Kolben 78a auf, der zu einer Befüllung zurückgezogen bzw. bei der Befüllung zurückgedrückt wird.

Bei einem alternativen Abfallbehälter 28k (Fig. 21) einer alternativen Vorrichtung 10k weist ein bewegbarer Kolben 78k ein Filter 80k auf, der zu einem Druckausgleich bei einer Befüllung vorgesehen ist. Der Abfallbehälter 28k ist mittels des Filters 80k für einen Druckausgleichsbetrieb ausgebildet. Das Filter 80k ist als hydrophobes Filter ausgebildet, in Abhängigkeit von einem verwendeten Medium in einer Analyse kann das Filter 80k auch als hydrophiles Filter ausgebildet sein.

Eine weitere alternative Vorrichtung 10l weist einen eingelegten Auffangbehälter 82l (Fig. 22) auf, der sich bei einer Befüllung (Fig. 23) ausdehnt. Zu einem Druckausgleich bei der Befüllung weist ein bewegbarer Kolben 78l ein hydrophobes Filter 80l auf. Der Abfallbehälter 28l ist mittels des hydrophoben Filters 80l für einen Druckausgleichsbetrieb ausgebildet

Ein alternativer Abfallbehälter 28m (Fig. 24) einer Vorrichtung 10m weist einen Dochtkörper 84m auf, der mit Absorbermaterial 86m gefüllt ist. Bei einer Befüllung des Abfallbehälters 28m wird Luft aus dem Dochtkörper 84m verdrängt und überschüssiges Flüssigkeitsvolumen von dem Absorbermaterial 86m gebunden. Das Absorbermaterial 86m kann beispielsweise von organischen Absorbern wie tropffreiem organischem Schwammmaterial, von kapillaren Kunststoffspeichern, wie sie beispielsweise von der Firma POREX hergestellt werden, von hygroskopischen Materialien wie beispielsweise Silikagel oder organischen Superabsorbermaterialien wie beispielsweise das unter dem Handelsnamen Luquasorb® von der Firma BASF vertriebene Produkt, gebildet sein. Anstelle des Absorbermaterials 86m kann auch ein Adsorbermaterial verwendet werden, wie beispielsweise gesinterte Kunststoffspeicher oder mineralische Adsorber wie getrocknete Tonmineralien oder Aktivkohle. In alternativen Ausgestaltungen kann der Dochtkörper 84m zu einer Aufnahme überschüssiger Flüssigkeitsvolumina vorgesehen sein. Der Abfallbehälter 28k weist einen hydrophoben Filter 80m in einem bewegbaren Kolben auf und ist für einen Druckausgleichsbetrieb ausgebildet.

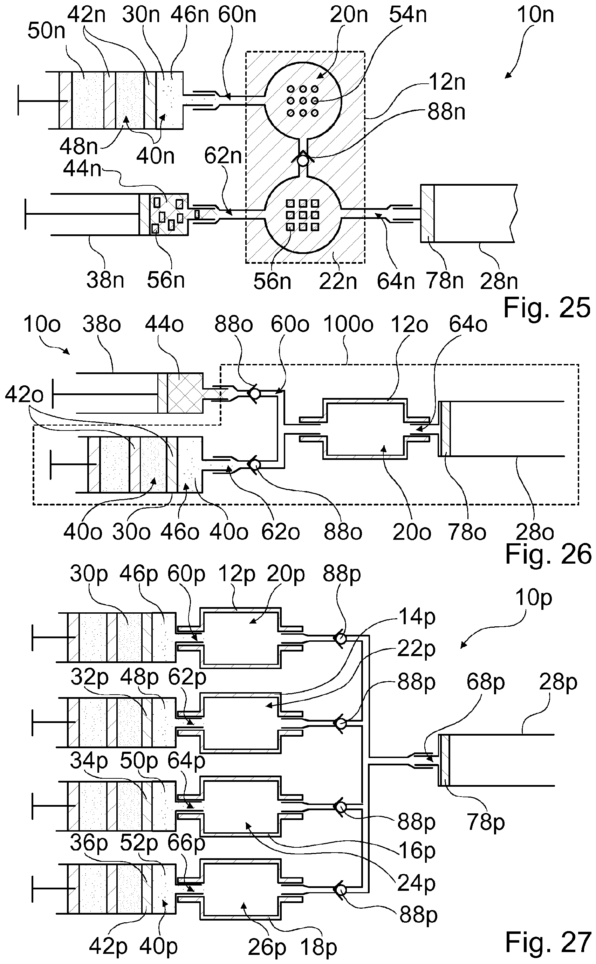

Eine alternative Vorrichtung 10n (Fig. 25) weist einen Reaktionsbehälter 12n mit zwei Arbeitsvolumina 20n, 22n auf, in denen Detektionsantikörper 54n bzw. Fängerantikörper 56n, vorzugsweise getrocknet, gebunden sind und die über eine Verbindung mit einem Ventil 88n verbunden sind. Die Arbeitsvolumina 20n, 22n sind über Schnittstellen 60n, 62n, 64n mit von einem Probenbehälter 38n, der eine Probe 44n mit magnetischen Mischungskörpern 58n gemischt enthält, von einem als Mehrkammerspritze ausgebildeten Analysematerialbehälter 30n mit mehreren Analysematerialien 46n, 48n, 50n in mehreren durch Trennelemente 42n unterteilte Teilkammern 40n und von einem als Abfallbehälter 28n mit einem verschiebbaren Kolben 78n ausgebildeten weiteren Medienbehältern verbunden.

In einer alternativen Vorrichtung 10o (Fig. 26) sind ein Reaktionsbehälter 12o, der ein Arbeitsvolumen 20o, ein Analysematerialbehälter 30o und ein Abfallbehälter 28o zu einem Modul 100o, das zu einer Verbindung mit einem als Probenbehälter 38o ausgebildeten weiteren Medienbehälter vorgesehen ist, vormontiert. Das Modul 100o ist über eine Schnittstelle 60o, das ein Ventil 88o aufweist, mit dem Probenbehälter 38o, der eine Probe 44o enthält, verbunden. Eine Schnittstelle 62o innerhalb des Moduls 100o, die das Arbeitsvolumen 20o mit dem als Mehrkammerspritze ausgebildeten Analysematerialbehälter 30o verbindet, weist ebenfalls ein Ventil 88o auf.

Eine alternative Vorrichtung 10p (Fig. 27) weist vier Reaktionsbehälter 12p, 14p, 16p, 18p mit Arbeitsvolumina 20p, 22p, 24p, 26p auf, die jeweils über eine Schnittstelle 60p, 62p, 64p, 66p mit als Mehrkammerspritzen ausgebildeten Analysematerialbehältern 30p, 32p, 34p, 36p verbunden sind. Die Arbeitsvolumina 20p, 22p, 24p, 26p sind über eine gemeinsame Schnittstelle 68p mit Ventilen 88p mit einem Abfallbehälter 28p mit einem verschiebbaren Kolben 78p verbunden. Die Vorrichtung 10p ist zu einer parallelen Durchführung mehrerer biochemischer Analysen vorgesehen. Es können in der Vorrichtung 10p parallel mehrere gleiche biochemische Analysen, beispielsweise eine Untersuchung derselben oder unterschiedlicher Proben auf denselben Analyten, und/oder mehrere unterschiedliche biochemische Analysen, beispielsweise eine Untersuchung mehrere Volumina einer Probe jeweils auf unterschiedliche Analyten, durchgeführt werden.

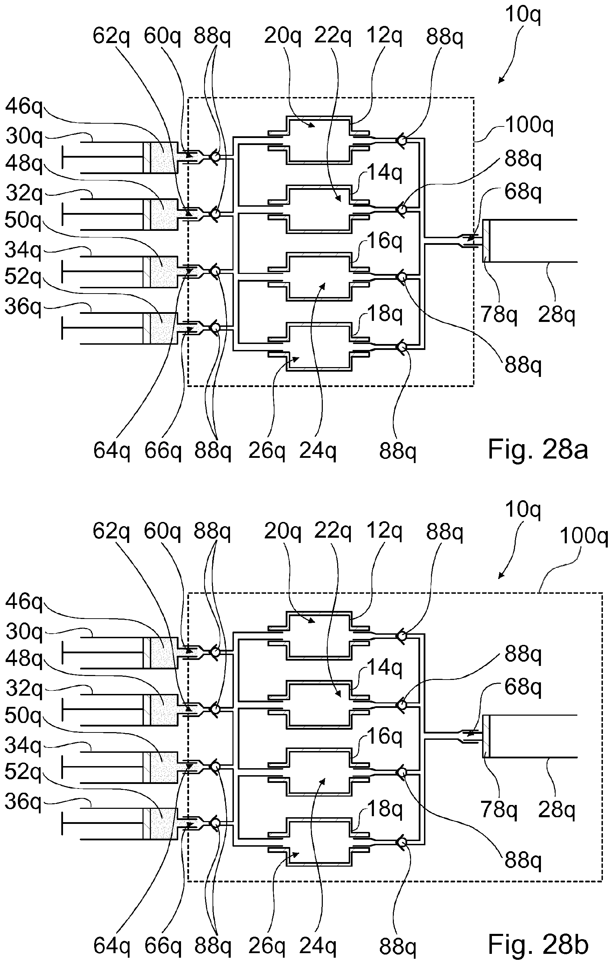

In einer weiteren alternativen Vorrichtung 10q (Fig. 28a) sind vier parallel zueinander angeordnete Reaktionsbehälter 12q, 14q, 16q, 18q mit Arbeitsvolumina 20q, 22q, 24q, 26q zu einem Modul 100q vormontiert, das dazu vorgesehen ist, eine parallele Durchführung mehrerer biochemischer Analysen zu ermöglichen. Das Modul 100q ist über Schnittstellen 60q, 62q, 64q, 66q, die Ventile 88q aufweisen, mit Analysematerialbehältern 30q, 32q, 34q, 36q verbunden, in denen jeweils ein Analysematerial 46q, 48q, 50q, 52q gelagert ist. Über eine gemeinsame Schnittstelle 68q mit Ventilen 88q sind die Arbeitsvolumina 20q, 22q, 24q, 26q mit einem Abfallbehälter 28q mit einem verschiebbaren Kolben 78q verbunden. In alternativen Ausgestaltungen kann das Modul 100q auch den Abfallbehälter 28q (Fig. 28b) und/oder einen oder mehrere der Analysematerialbehälter 30q, 32q, 34q, 36q zusätzlich umfassen.

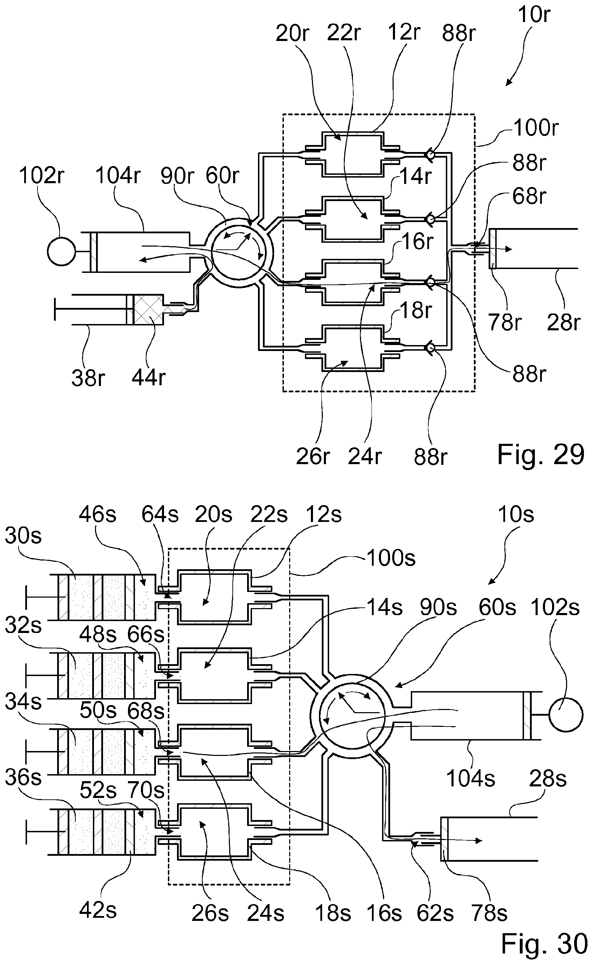

In einer weiteren alternativen Vorrichtung 10r (Fig. 29) sind ebenfalls vier parallel zueinander angeordnete Reaktionsbehälter 12r, 14r, 16r, 18r mit Arbeitsvolumina 20r, 22r, 24r, 26r zu einem Modul 100r vormontiert, das dazu vorgesehen ist, eine sequentielle oder teilweise parallele Durchführung mehrerer biochemischer Analysen zu ermöglichen. Eine Beschickung der Reaktionsbehälter 12r, 14r, 16r, 18r erfolgt über eine Schnittstelle 60r, die ein Multiportventil 90r aufweist. Das Multiportventil 90r ist dazu ausgebildet, eine Probe 44r aus einem Probenbehälter 38r in eine Motorspritze 104r, die mit einem Motor 102r gekoppelt, zu überführen. Mittels des Motors 102r wird die Probe 44r aus der Motorspritze 104r mittels Überdruck abgegeben und mit dem Multiportventil 90r an eines der Arbeitsvolumina 20r, 22r, 24r, 26r geschickt. Grundsätzlich kann das Multiportventil 90r mit weiteren Probenbehältern 38r oder mit weiteren Medienbehältern gekoppelt sein. Zu einer Weiterführung der biochemischen Analyse wird der Probenbehälter 38r gegen einen Medienbehälter mit weiteren Materialien für die biochemische Analyse ausgetauscht, die ebenfalls über das Multiportventil 90r und die Motorspritze 104r abgegeben werden. Der Probenbehälter 38r kann alternativ als Mehrkammerspritze ausgebildet sein und weitere Reagenzien für die biochemische Analyse lagern. Eine Schnittstelle 62r verbindet das Modul 100r mit einem Abfallbehälter 28r, der in alternativen Ausbildungen auch in dem Modul 100r eingeschlossen sein kann.

In einer weiteren alternativen Vorrichtung 10s (Fig. 30) sind ebenfalls vier parallel zueinander angeordnete Reaktionsbehälter 12s, 14s, 16s, 18s mit Arbeitsvolumina 20s, 22s, 24s, 26s zu einem Modul 100s vormontiert, das dazu vorgesehen ist, eine sequentielle oder teilweise parallele Durchführung mehrerer biochemischer Analysen zu ermöglichen. Die Arbeitsvolumina 20s, 22s, 24s, 26s sind über Schnittstellen 64s, 66s, 68s, 70s mit als Mehrkammerspritzen, die mit Trennelementen 42s abgetrennte Teilkammer 40s aufweisen, ausgebildeten Analysematerial-behältern 30s, 32s, 34s, 36s verbunden. Eine den vier Arbeitsvolumina 20s, 22s, 24s, 26s gemeinsame Schnittstelle 60s weist ein Multiportventil 90s auf, das an eine Motorspritze 104s, die mit einem Motor 102s gekoppelt ist, angeschlossen ist. Über das Multiportventil 90s und die Motorspritze 104s werden Flüssigkeitsvolumina aus Medienbehälter mittels Unterdruck angesaugt und gezielt in einzelne Arbeitsvolumina 20s, 22s, 24s, 26s eingebracht. Überschüssige Flüssigkeitsvolumina werden gezielt aus den Arbeitsvolumina 20s, 22s, 24s, 26s angesaugt und in die Motorspritze 104s überführt. Mittels des Motors 102s wird anschließend das überschüssige Flüssigkeitsvolumen aus der Motorspritze 104r mit Überdruck ausgestoßen und mit dem Multiportventil 90r in einen Abfallbehälter 28s mit verschiebbarem Kolben 78s verschickt. In alternativen Ausbildungen kann der Abfallbehälter 28s auch in dem Modul 100s eingeschlossen sein.

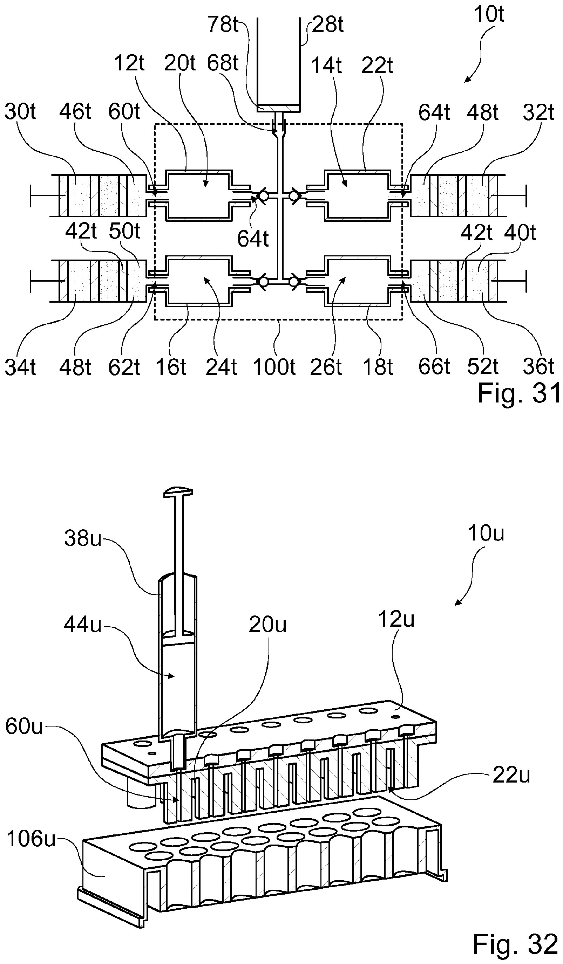

In einer weiteren alternativen Vorrichtung 10t (Fig. 31) sind vier Reaktionsbehälter 12t, 14t, 16t, 18t mit Arbeitsvolumina 20t, 22t, 24t, 26t in einer zweireihigen Anordnung zu einem Modul 100t vormontiert, das dazu vorgesehen ist, eine parallele Durchführung mehrerer biochemischer Analysen zu ermöglichen. Über eine gemeinsame Schnittstelle 68t mit Ventilen 88t sind die Arbeitsvolumina 20t, 22t, 24t, 26t mit einem gemeinsamen Abfallbehälter 28t mit verschiebbarem Kolben 78t verbunden. Über Schnittstellen 60t, 62t, 64t, 66t sind die Arbeitsvolumina 20t, 22t, 24t, 26t mit als Mehrkammerspritzen ausgebildeten Analysematerialbehältern 30t, 32t, 34t, 36t verbunden. In alternativen Ausgestaltungen können der Abfallbehälter 28t und/oder einer oder mehrere der Analysematerialbehältern 30t, 32t, 34t, 36t in dem Modul 100t vormontiert sein.

In Fig. 32 ist ein Reaktionsbehälter 12u einer alternativen Vorrichtung 10u dargestellt, der als ein Verbindungsblock zu einer Verbindung mit einer kommerziellen Multiwell-Platte 106u vor-gesehen ist, und der eine Vielzahl von Arbeitsvolumina 20u, 22u (aus Gründen der Übersichtlichkeit sind weitere Arbeitsvolumina unbezeichnet belassen) aufweist. Durch die Verbindung werden einzelne Wells der Multiwell-Platte 106u als Bodenelemente der Arbeitsvolumina 20u, 22u genutzt und ergänzen den Reaktionsbehälter 12u zu einem im Wesentlichen vollständig geschlossenen Gefäß. Schnittstellen 60u verbinden die Arbeitsvolumina 20u, 22u mit weiteren Medienbehältern wie einem Probenbehälter 38u, der eine Probe 44u lagert, oder einem Abfallbehälter (hier nicht dargestellt).

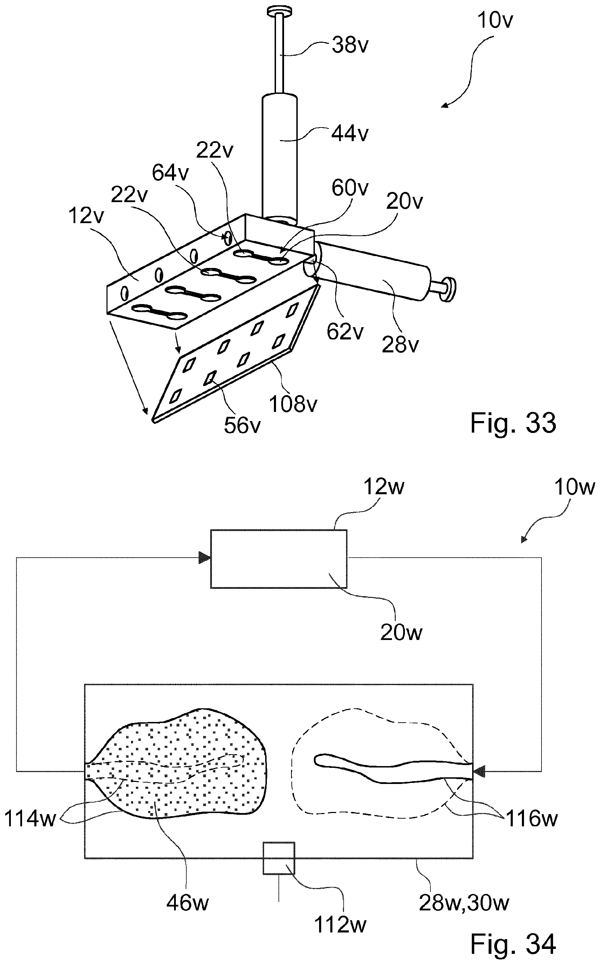

Fig. 33 zeigt einen Reaktionsbehälter 12v einer alternativen Vorrichtung 10v mit einer Vielzahl von Arbeitsvolumina 20v, 22v, der mit einem kommerziellen Planararray 108v mit daran in Punktform gebundenen Fängerantikörpern 56v zu einem im Wesentlichen vollständig geschlossenen Gefäß montiert wird. Eine Montage kann form- und/oder kraftschlüssig beispielsweise durch Kleben oder Schweißen erfolgen. Schnittstellen 60v, 62v, 64v sind zu einer Verbindung der Arbeitsvolumina 20v, 22v mit Probenbehältern 38v, die eine Probe 44v lagern, mit Abfallbehältern 28v und/oder mit weiteren Medienbehältern vorgesehen.

Fig. 34 zeigt eine alternative Vorrichtung 10w mit einem Reaktionsbehälter 12w, der ein Arbeitsvolumen 20w aufweist, und mit einem Analysematerialbehälter 30w, der integriert mit einem Abfallbehälter 28w ausgeführt ist. Der integriert mit einem Abfallbehälter 28w ausgeführte Analysematerialbehälter 30w weist ein Kompartiment 114w zu einer Aufnahme von Analysematerialien 46w und ein Kompartiment 116w zu einer Aufnahme überschüssiger Flüssigkeitsvolumina auf, die beide als elastische Aufnahmesäcke ausgebildet sind, wobei vor einem Beginn einer Analysereaktion das Kompartiment 116w zur Aufnahme überschüssiger Flüssigkeitsvolumina leer vorliegt und zusammengefaltet ist. Aufgrund einer Entleerung des Kompartiments 114w für die Analysematerialien 46w im Zuge einer Durchführung einer biochemischen Analyse kann das Kompartiment zur Aufnahme überschüssiger Flüssigkeitsvolumina 116w sich bei Auffüllung ausdehnen. Gestrichelt dargestellt ist ein Zustand des integriert mit einem Abfallbehälter 28w ausgeführten Analysematerialbehälters 30w nach Durchführung der Analyse mit geleertem Kompartiment 114w für die Analysematerialien 46w und gefülltem Kompartiment 116w zur Aufnahme überschüssiger Flüssigkeitsvolumina. Es wird eine volumenneutrale Lagerung erreicht. Der Analysematerialbehälter 30w weist ein Ventil 112w zu einer Entlüftung auf, um einen druckneutralen Betrieb zu erreichen. Eine Entleerung des Kompartiments 114w für die Analysematerialien 46w wird durch eine Absaugung erreicht, in alternativen Ausgestaltungen kann eine Entleerung beispielsweise durch einen verschiebbaren Kolben, der einen Druck auf das Kompartiment 114w für die Analysematerialien 46w ausübt, erreicht werden. In alternativen Ausgestaltungen können die Kompartimente 114w, 116w beispielsweise verschiebbare Abschlusselemente zu einer Veränderung ihrer Volumina oder feste Volumina aufweisen, anstatt als elastische Aufnahmesäcke ausgebildet zu sein.

**Bezugszeichen**

| | | | |
|---|---|---|---|
| 10 | Vorrichtung | 84 | Dochtkörper |
| 12 | Reaktionsbehälter | 86 | Absorbermaterial |
| 14 | Reaktionsbehälter | 88 | Ventil |
| 16 | Reaktionsbehälter | 90 | Multiportventil |
| 18 | Reaktionsbehälter | 92 | O-Ring |
| 20 | Arbeitsvolumen | 94 | Magnetträgerkörper |
| 22 | Arbeitsvolumen | 96 | Träger |
| 24 | Arbeitsvolumen | 98 | Suspension |
| 26 | Arbeitsvolumen | 100 | Modul |
| 28 | Abfallbehälter | 102 | Motor |
| 30 | Analysematerialbehälter | 104 | Motorspritze |
| 32 | Analysematerialbehälter | 106 | Multiwell-Platte |
| 34 | Analysematerialbehälter | 108 | Planararray |
| 36 | Analysematerialbehälter | 110 | Magneteinheit |
| 38 | Probenbehälter | 112 | Ventil |
| 40 | Teilkammer | 114 | Kompartiment |
| 42 | Trennelemente | 116 | Kompartiment |
| 44 | Probe | | |
| 46 | Analysematerial | | |
| 48 | Analysematerial | | |
| 50 | Analysematerial | | |
| 52 | Analysematerial | | |
| 54 | Detektionsantikörper | | |
| 56 | Fängerantikörper | | |
| 58 | Mischungskörper | | |
| 60 | Schnittstelle | | |
| 62 | Schnittstelle | | |
| 64 | Schnittstelle | | |
| 66 | Schnittstelle | | |
| 68 | Schnittstelle | | |
| 70 | Schnittstelle | | |
| 72 | Grundkörper | | |
| 74 | Boden | | |
| 76 | Deckel | | |
| 78 | Kolben | | |
| 80 | Filter | | |
| 82 | Auffangbehälter | | |

## Patentansprüche

1. Vorrichtung zur Durchführung einer biochemischen Analyse, insbesondere im Weltraum, insbesondere eines Immunoassays, bei der zumindest ein Analyt in einer Probe (44a; 44n-o; 44r; 44u; 44v) selektiv bestimmt wird, mit zumindest einem Reaktionsbehälter (12a-j; 12n-w, 14a; 14p-t, 16p-t, 18p-t), der zumindest ein Arbeitsvolumen (20a-j; 20n-w, 22a; 22n; 22p-v, 24p-t, 26p-t) aufweist, das zu einer Aufnahme eines Flüssigkeitsvolumens und zur Durchführung zumindest eines Teilschritts einer Analysereaktion vorgesehen ist, und mit zumindest einer Schnittstelle (60a-j; 60n-v, 62a-j; 62n-t; 62v, 64a; 64d; 64n-q; 64s-t; 64v, 66a; 66o-q; 66s-t, 68o-q; 68s-t, 70s), die zu einer Verbindung zumindest eines Arbeitsvolumens (20a-j; 20n-w, 22a; 22n; 22p-v, 24p-t, 26p-t) mit einem weiteren Medienbehälter (28a; 28k-l; 28n-t; 28v-w, 30a; 30n-q; 30s-t; 30w, 32o-q; 32s-t, 34 o-q; 34s-t, 36o-q; 36s-t, 38a; 38n-o; 38r; 38u-v) vorgesehen ist,
wobei der Reaktionsbehälter (12a-j; 12n-w, 14a; 14p-t, 16p-t, 18p-t) als im montierten Zustand zumindest im Wesentlichen vollständig abgeschlossenes Gefäß ausgebildet ist, und mit zumindest einem als Abfallbehälter (28m) ausgebildeten weiteren Medienbehälter, der zu einer Aufnahme überschüssiger Flüssigkeitsvolumina vorgesehen ist,
**dadurch gekennzeichnet, dass**
der Abfallbehälter (28m) für einen Druckausgleichsbetrieb ein hydrophobes oder hydrophiles Filter (80m) für einen Druckausgleich mit einer Umgebung aufweist und dass der Abfallbehälter (28m) zumindest einen Dochtkörper (84m) aufweist, der mit Absorbermaterial (86m) gefüllt ist, das von tropffreiem organischem Schwammmaterial, kapillaren Kunststoffspeichern oder einem organischen Superabsorbermaterial, wie beispielsweise dem unter dem Handelsnamen Luquasorb® von der Firma BASF vertriebenen Produkt, gebildet ist.

2. Vorrichtung nach Anspruch 1,
**dadurch gekennzeichnet, dass**
das Arbeitsvolumen (20a-j; 20n-w, 22a; 22n; 22p-v, 24p-t, 26p-t) für eine Reaktionsdurchführung unter Bedingungen reduzierter Schwerkraft ausgebildet ist.

3. Vorrichtung nach Anspruch 2,
**dadurch gekennzeichnet, dass**
das Arbeitsvolumen (20a-j; 20n-w, 22a; 22n; 22p-v, 24p-t, 26p-t) eine sich von einer Schnittstelle (60a-j; 60n-v, 62a-j; 62n-t; 62v, 64a; 64d; 64n-q; 64s-t; 64v, 66a; 66o-q; 66s-t, 68o-q; 68s-t, 70s) aus aufweitende Form aufweist.

4. Vorrichtung nach Anspruch 3,
**dadurch gekennzeichnet, dass**
das Arbeitsvolumen (20b) zumindest im Wesentlichen rechteckförmig ausgebildet ist.

5. Vorrichtung nach Anspruch 3,
**dadurch gekennzeichnet, dass**
das Arbeitsvolumen (20c) in einer Tropfen-Form ausgebildet ist.

6. Vorrichtung nach Anspruch 3,
**dadurch gekennzeichnet, dass**
das Arbeitsvolumen (20a; 20e-j; 20n-v, 22a; 22n; 22p-v, 24p-t, 26p-t) in einer Kreis-Form ausgebildet ist.

7. Vorrichtung nach Anspruch 3,
**dadurch gekennzeichnet, dass**
das Arbeitsvolumen (20d) in einer Düsen-Form ausgebildet ist.

8. Vorrichtung nach einem der vorhergehenden Ansprüche,
**dadurch gekennzeichnet, dass**
der Abfallbehälter (28a; 28k-l; 28n-t; 28v-w) zumindest im Wesentlichen vollständig abgeschlossen ausgebildet ist.

9. Vorrichtung nach einem der vorhergehenden Ansprüche,
**gekennzeichnet durch**
zumindest einen als Analysematerialbehälter (30a; 30n-q; 30s-t; 30w, 32o-q; 32s-t, 34 o-q; 34s-t, 36 o-q; 36s-t), der zu einer Bereitstellung von Analysematerialien (46a; 46n-q; 46s-t; 46w, 48a; 48o-q; 48s-t, 50a; 50o-q; 50s-t, 52a; 52o-q; 52s-t) vorgesehen ist, ausgebildeten weiteren Medienbehälter.

10. Vorrichtung nach Anspruch 9,
**dadurch gekennzeichnet, dass**
der Analysematerialbehälter (30a; 30n-q; 30s-t, 32o-q; 32s-t, 34 o-q; 34s-t, 36 o-q; 36s-t) als Mehrkammerspritze ausgebildet ist.

11. Vorrichtung nach Anspruch 9 oder 10,
**dadurch gekennzeichnet, dass**
der Analysematerialbehälter (30w) integriert mit einem Abfallbehälter (28w) ausgebildet ist.

12. Vorrichtung nach einem der vorhergehenden Ansprüche,
**dadurch gekennzeichnet, dass**
zumindest ein Reaktionsbehälter (12o; 12q-t) zusammen mit zumindest einem weiteren Reaktionsbehälter (12q-t) und/oder zumindest einem weiteren Medienbehälter (28o, 30o) zu einem Modul (100o; 100q-t), das zu einer Verbindung mit einem weiteren Medienbehälter (28q-t, 30q; 30s-t, 32s-t, 34s-t, 36s-t, 38o) vorgesehen ist, vormontiert ist.

13. Vorrichtung nach Anspruch 12,
**dadurch gekennzeichnet, dass**
das Modul (100q-t) dazu vorgesehen ist, eine parallele Durchführung mehrerer biochemischer Analysen zu ermöglichen.

14. Vorrichtung nach einem der vorhergehenden Ansprüche,
**gekennzeichnet durch**
magnetische Mischungskörper (58a; 58n), die zu einer Durchmischung von Reaktionsmaterialien (46a; 46n-q; 46s-t, 48a; 48o-q; 48s-t, 50a; 50o-q; 50s-t, 52a; 52o-q; 52s-t) und der Probe (44a; 44n-o; 44r; 44u; 44v) für eine Analysereaktion vorgesehen sind.

15. Vorrichtung nach einem der vorhergehenden Ansprüche,
**dadurch gekennzeichnet, dass**
der Reaktionsbehälter (12u) zu einer Verbindung mit einer kommerziellen Multiwell-Platte (106u) vorgesehen ist.

16. Vorrichtung nach einem der vorhergehenden Ansprüche,
**dadurch gekennzeichnet, dass**
der Reaktionsbehälter (12v) zu einer Verbindung mit einem kommerziellen Planararray (108v) vorgesehen ist.

17. Verfahren zur Durchführung einer biochemischen Analyse mit einer Vorrichtung (10a-v) nach einem der Ansprüche 1 - 16.

18. Verfahren nach Anspruch 17,
**dadurch gekennzeichnet, dass**
die Durchführung unter Bedingungen reduzierter Schwerkraft erfolgt.

19. Verfahren nach Anspruch 17 oder 18,
**dadurch gekennzeichnet, dass**
eine Durchmischung von Analysematerialien (46a; 46n-q; 46s-t; 46w, 48a; 48o-q; 48s-t, 50a; 50o-q; 50s-t, 52a; 52o-q; 52s-t) und der Probe (44a; 44n-o; 44r; 44u; 44v) für die Analysereaktion mittels magnetischer Mischungskörper (58a; 58n) erfolgt.

20. Verfahren zumindest nach Anspruch 17,
**dadurch gekennzeichnet, dass**
an einer Analysereaktion nur Reaktionsmaterialien (46a; 46n-q; 46s-t; 46w, 48a; 48o-q; 48s-t, 50a; 50o-q; 50s-t, 52a; 52o-q; 52s-t) und Proben (44a; 44n-o; 44r; 44u; 44v) innerhalb eines Arbeitsvolumens eines Reaktionsbehälters beteiligt sind.

21. Verfahren zumindest nach Anspruch 17,
**dadurch gekennzeichnet, dass**
eine Zugabe von Reaktionsmaterialien (46a; 46n-q; 46s-t; 46w, 48a; 48o-q; 48s-t, 50a; 50o-q; 50s-t, 52a; 52o-q; 52s-t) und Proben (44a; 44n-o; 44r; 44u; 44v) in beliebig kleinen Subvolumina und mit eingelegten Pausen ablaufen kann.

## Claims

1. Device for performing a biochemical analysis, in particular in outer space, in particular an immunoassay, in which at least one analyte in a sample (44a; 44n-o; 44r; 44u; 44v) is determined selectively, with at least one reaction vessel (12a-j; 12n-w, 14a; 14p-t, 16p-t, 18p-t) having at least one work volume (20a-j; 20n-w, 22a; 22n; 22p-v, 24p-t, 26p-t) that is provided for taking in a liquid volume and for performing at least one sub-step of an analysis reaction, and with at least one interface (60a-j; 60n-v, 62a-j; 62n-t; 62v, 64a; 64d; 64n-q; 64s-t; 64v, 66a; 66o-q; 66s-t, 68o-q; 68s-t, 70s) that is provided for a connection of at least one work volume (20a-j; 20n-w, 22a; 22n; 22p-v, 24p-t, 26p-t) to a further media container (28a; 28k-l; 28n-t; 28v-w, 30a; 30n-q; 30s-t; 30w, 32o-q; 32s-t, 34o-q; 34s-t, 36o-q; 36s-t, 38a; 38n-o; 38r; 38u-v), the reaction vessel (12a-j; 12n-w, 14a; 14p-t, 16p-t, 18p-t) being embodied as a vessel which is at least substantially closed-off in an assembled state, and with at least one additional media container which is embodied as a waste container (28m) and is provided for taking in excess liquid volumes,
**characterised in that**
for a pressure-equalisation operation the waste container (28m) comprises a hydrophobic or hydrophilic filter (80m) for a pressure equalisation with an environment, and that the waste container (28m) comprises at least one wick body (84m), which is filled with absorber material (86m) implemented by drip-free organic sponge material, by capillary synthetic storage materials or by an organic superabsorber material, e.g. the product distributed by BASF under the trade name Luquasorb®.

2. Device according to claim 1,
**characterised in that**
the work volume (20a-j; 20n-w, 22a; 22n; 22p-v, 24p-t, 26p-t) is implemented for reaction performance under low-gravity conditions.

3. Device according to claim 2,
**characterised in that**
the work volume (20a-j; 20n-w, 22a; 22n; 22p-v, 24p-t, 26p-t) has a shape that widens starting from an interface (60a-j; 60n-v, 62a-j; 62n-t; 62v, 64a; 64d; 64n-q; 64s-t; 64v, 66a; 66o-q; 66s-t, 68o-q; 68s-t, 70s).

4. Device according to claim 3,
**characterised in that**
the work volume (20b) is embodied at least substantially rectangular.

5. Device according to claim 3,
**characterised in that**
the work volume (20c) is embodied drop-shaped.

6. Device according to claim 3,
**characterised in that**
the work volume (20a; 20e-j; 20n-v, 22a; 22n; 22p-v, 24p-t, 26p-t) is embodied circle-shaped.

7. Device according to claim 3,
**characterised in that**
the work volume (20d) is embodied nozzle-shaped.

8. Device according to one of the preceding claims,
**characterised in that**
the waste container (28a; 28k-l; 28n-t; 28v-w) is embodied at least substantially entirely closed off.

9. Device according to one of the preceding claims,
**characterised by**
at least one further media container, which is embodied as an analysis-material container (30a; 30n-q; 30s-t; 30w, 32o-q; 32s-t, 34o-q; 34s-t, 36o-q; 36s-t) provided for supplying analysis materials (46a; 46n-q; 46s-t; 46w, 48a; 48o-q; 48s-t, 50a; 50o-q; 50s-t, 52a; 52o-q; 52s-t).

10. Device according to claim 9,
**characterised in that**
the analysis-material container (30a; 30n-q; 30s-t; 32o-q; 32s-t, 34o-q; 34s-t, 36o-q; 36s-t) is embodied as a multi-chamber syringe.

11. Device according to claim 9 or 10,
**characterised in that**
the analysis-material container (30w) is embodied integrally with a waste container (28w).

12. Device according to one of the preceding claims,
**characterised in that**
at least one reaction vessel (12o; 12q-t), is pre-assembled together with at least one further reaction vessel (12q-t) and/or at least one further media container (28o, 30o) to implement a module (100o; 100q-t) which is provided for a connection to a further media container (28q-t, 30q; 30s-t, 32s-t, 34s-t, 36s-t, 38o).

13. Device according to claim 12,
**characterised in that**
the module (100q-t) is provided to allow a plurality of biochemical analyses to be carried out in parallel.

14. Device according to one of the preceding claims,
**characterised by**
magnetic mixing bodies (58a; 58n), which are provided for mixing reaction materials (46a; 46n-q; 46s-t, 48a; 48o-q; 48s-t, 50a; 50o-q; 50s-t, 52a; 52o-q; 52s-t) and the sample (44a; 44n-o; 44r; 44u; 44v) for an analytic reaction.

15. Device according to one of the preceding claims,
**characterised in that**
the reaction vessel (12u) is provided for a connection to a commercial Multiwell plate (106u).

16. Device according to one of the preceding claims,
**characterised in that**
the reaction vessel (12v) is provided for a connection to a commercial planar array (108v).

17. Method for performing a biochemical analysis with a device (10a-v) according to one of claims 1 to 16.

18. Method according to claim 17,
**characterised in that**
the performance is effected under reduced-gravity conditions.

19. Method according to claim 17 or 18,
**characterised in that**
a mixing of analysis materials (46a; 46n-q; 46s-t; 46w, 48a; 48o-q; 48s-t, 50a; 50o-q; 50s-t, 52a; 52o-q; 52s-t) and the sample (44a; 44n-o; 44r; 44u; 44v) for the analysis reaction is effected by means of magnetic mixing bodies (58a; 58n).

20. Method at least according to claim 17,
**characterised in that**
only reaction materials (46a; 46n-q; 46s-t; 46w, 48a; 48o-q; 48s-t, 50a; 50o-q; 50s-t, 52a; 52o-q; 52s-t) and samples (44a; 44n-o; 44r; 44u; 44v) within a work volume of a reaction vessel are involved in an analysis reaction.

21. Method at least according to claim 17,
**characterised in that**
an addition of reaction materials (46a; 46n-q; 46s-t; 46w, 48a; 48o-q; 48s-t, 50a; 50o-q; 50s-t, 52a; 52o-q; 52s-t) and samples (44a; 44n-o; 44r; 44u; 44v) may be effected in sub-volumina that are as small as desired, and with pauses in between.

## Revendications

1. Appareil pour la réalisation d'une analyse biochimique, en particulier dans l'espace, en particulier d'un immunoassay, dans laquelle au moins un analyte d'un échantillon (44a ; 44n-o ; 44r ; 44u ; 44v) est identifié sélectivement, avec au moins une cuve à réaction (12a-j ; 12n-w, 14a ; 14p-t, 16p-t, 18p-t), laquelle comporte au moins un volume opératif (20a-j ; 20n-w, 22a ; 22n ; 22p-v, 24p-t, 26p-t) prévu à recevoir un volume liquide et à réaliser au moins un étape partiel d'une réaction analytique, et avec au moins un interface (60a-j ; 60n-v, 62a-j ; 62n-t ; 62v, 64a ; 64d ; 64n-q ; 64s-t ; 64v, 66a ; 66o-q ; 66s-t, 68o-q ; 68s-t, 70s) prévu à une connexion d'au moins un volume opératif (20a-j ; 20n-w, 22a ; 22n ; 22p-v, 24p-t, 26p-t) à un autre récipient de media (28a ; 28k-l ; 28n-t ; 28v-w, 30a ; 30n-q ; 30s-t ; 30w, 32o-q ; 32s-t, 34o-q ; 34s-t, 36o-q ; 36s-t, 38a ; 38n-o ; 38r ; 38u-v),- 1-la cuve á réaction (12a-j ; 12n-w, 14a ; 14p-t, 16p-t, 18p-t) étant implémentée comme une cuve au moins substantiellement entièrement close dans l'état assemblé, et avec au moins un autre récipient de media implémenté comme récipient à déchets (28m) et prévu à recevoir des volumes liquides de surplus,
**caractérisé en ce que**
pour une opération de pressurisation le récipient à déchets (28m) comporte un filtre (80m) hydrophobe ou hydrophile pour une compensation de pressure avec un environnement, et que le récipient à déchets (28m) comporte au moins un corps de mèche (84m), lequel est rempli de matériau absorbant (86m) implémenté par de matériau organique spongieux et non-gouttant, des réservoirs capillaires de plastique ou d'un matériau organique super-absorbant, par exemple le produit distribué par la compagnie BASF sous le nom commercial Luquasorb®.

2. Appareil selon la revendication 1,
**caractérisé en ce que**
le volume opératif (20a-j ; 20n-w, 22a ; 22n ; 22p-v, 24p-t, 26p-t) est implémente pour une réalisation de réaction sous conditions de gravitation réduite.

3. Appareil selon la revendication 2,
**caractérisé en ce que**
le volume opératif (20a-j ; 20n-w, 22a ; 22n ; 22p-v, 24p-t, 26p-t) comporte une forme s'agrandissant à partir d'un interface (60a-j ; 60n-v), 62a-j ; 62n-t ; 62v, 64a ; 64d ; 64n-q ; 64s-t ; 64v, 66a ; 66o-q ; 66s-t, 68o-q ; 68s-t, 70s).

4. Appareil selon la revendication 3,
**caractérisé en ce que**
le volume opératif (20b) est implémenté au moins substantiellement en forme rectangulaire.

5. Appareil selon la revendication 3,
**caractérisé en ce que**
le volume opératif (20c) est implémenté en forme de goutte.

6. Appareil selon la revendication 3,
**caractérisé en ce que**
le volume opératif (20a ; 20e-j ; 20n-v, 22a ; 22n ; 22p-v, 24p-t, 26p-t) est implémenté en forme circulaire.

7. Appareil selon la revendication 3,
**caractérisé en ce que**
le volume opératif (20d) est implémenté en forme de gicleur.

8. Appareil selon l'une quelconque des revendications précédentes,
**caractérisé en ce que**
le récipient à déchets (28a ; 28k-l ; 28n-t ; 28v-w) est implémenté au moins substantiellement entièrement clos.

9. Appareil selon l'une quelconque des revendications précédentes,
**caractérisé par**
au moins un autre récipient de media implémenté comme récipient de matériau analytique (30a ; 30n-q ; 30s-t ; 30w, 32o-q ; 32s-t, 34o-q ; 34s-t, 36o-q ; 36s-t) prévu à fournir des matériaux analytiques (46a ; 46n-q ; 46s-t ; 46w, 48a ; 48o-q ; 48s-t, 50a ; 50o-q ; 50s-t, 52a ; 52o-q ; 52s-t).

10. Appareil selon la revendication 9,
**caractérisé en ce que**
le récipient de matériau analytique (30a ; 30n-q ; 30s-t ; 32o-q ; 32s-t, 34o-q ; 34s-t, 36o-q ; 36s-t) est implémenté comme seringue à plusieurs chambres.

11. Appareil selon la revendication 9 ou 10,
**caractérisé en ce que**
le récipient de matériau analytique (30w) est implémenté intégralement avec un récipient à déchets (28w).

12. Appareil selon l'une quelconque des revendications précédentes,
**caractérisé en ce qu'**
au moins une cuve á réaction (12o ; 12q-t), ensemble avec au moins une autre cuve á réaction (12q-t) et/ou avec au moins un autre récipient de média (28o, 30o), est préassemblé pour former un module (100o ; 100q-t), lequel est prévu pour une connexion à un autre récipient de média (28q-t, 30q ; 30s-t, 32s-t, 34s-t, 36s-t, 38o).

13. Appareil selon la revendication 12,
**caractérisé en ce que**
le module (100q-t) est prévu à permettre une réalisation parallèle de plusieurs analyses biochimiques.

14. Appareil selon l'une quelconque des revendications précédentes,
**caractérisé par**
des corps mélangeurs magnétiques (58a ; 58n) prévus à mélanger des matériaux de réaction (46a ; 46n-q ; 46s-t, 48a ; 48o-q ; 48s-t, 50a ; 50o-q ; 50s-t, 52a ; 52o-q ; 52s-t) et l'échantillon (44a ; 44n-o ; 44r ; 44u ; 44v) pour une réaction analytique.

15. Appareil selon l'une quelconque des revendications précédentes,
**caractérisé en ce que**
la cuve à réaction (12u) est prévue à une connexion avec une plaque Multiwell (106u) commerciale.

16. Appareil selon l'une quelconque des revendications précédentes,
**caractérisé en ce que**
la cuve à réaction (12v) est prévue à une connexion avec un array planaire (108v) commercial.

17. Procédé pour réaliser une analyse biochimique par le biais d'un appareil (10a-v) selon l'une quelconque des revendications 1 à 16.

18. Procédé selon la revendication 17,
**caractérisé en ce que**
la réalisation est effectuée sous conditions de gravitation réduite.

19. Procédé selon la revendication 17 ou 18,
**caractérisé en ce qu'**
une mélange de matériaux analytiques (46a ; 46n-q, 46s-t ; 46w, 48a ; 48o-q ; 48s-t, 50a ; 50o-q ; 50s-t, 52a ; 52o-q ; 52s-t) et de l'échantillon (44a ; 44n-o ; 44r ; 44u ; 44v) est effectuée pour la réaction analytique par le biais des corps mélangeurs magnétiques (58a ; 58n).

20. Procédé au moins selon la revendication 17,
**caractérisé en ce que**
seulement des matériaux à réaction (46a ; 46n-q ; 46s-t ; 46w, 48a ; 48o-q ; 48s-t, 50a ; 50o-q ; 50s-t, 52a ; 52o-q ; 52s-t) et des échantillons (44a ; 44n-o ; 44r ; 44u ; 44v) à l'intérieur d'un volume opératif d'une cuve à réaction sont parties d'une réaction analytique.

21. Procédé au moins selon la revendication 17,
**caractérisé en ce qu'**
une addition des matériaux à réaction (46a ; 46n-q ; 46s-t ; 46w, 48a ; 48o-q ; 48s-t, 50a ; 50o-q ; 50s-t, 52a ; 52o-q ; 52s-t) et des échantillons (44a ; 44n-o ; 44r ; 44u ; 44v) peut se dérouler avec des sous-volumes d'une petitesse quelconque et faisant des pauses.
